(19) Europäisches Patentamt
European Patent Office
Office européen des brevets

(11) EP 3 034 538 A1

(12) EUROPEAN PATENT APPLICATION

(43) Date of publication:
**22.06.2016 Bulletin 2016/25**

(51) Int Cl.:
***C08G 65/22*** *(2006.01)* ***A61K 47/48*** *(2006.01)*
***C08F 32/04*** *(2006.01)*

(21) Application number: **14307083.7**

(22) Date of filing: **18.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **UNIVERSITE DE BORDEAUX**
  **33000 Bordeaux (FR)**
- **Institut Polytechnique De Bordeaux (IPB)**
  **33400 Talence (FR)**
- **Centre National de la Recherche Scientifique**
  **75016 Paris (FR)**

(72) Inventors:
- **Durrieu, Marie-Christine**
  **33000 BORDEAUX (FR)**
- **Heroguez, Valérie**
  **33700 MERIGNAC (FR)**
- **Pichavant, Loïc**
  **33000 BORDEAUX (FR)**
- **Carrie, Hélène**
  **33124 BRANNENS (FR)**

(74) Representative: **Tezier Herman, Béatrice et al**
**Cabinet Becker & Associés**
**25, rue Louis le Grand**
**75002 Paris (FR)**

# (54) Polymer particles and biomaterials comprising the same

(57) The present invention relates to polymer particles comprising antibiotics which are deliverable *in situ,* as well as a method of preparation thereof. The present invention also relates to bioactive biomaterials for the controlled delivery of antibiotics comprising support materials having such polymer particles on their surface. The invention also relates to implants, prostheses, stents, lenses or cements as well as any pharmaceutical composition comprising said biomaterials.

EP 3 034 538 A1

## Description

### FIELD OF THE INVENTION

The present invention relates to polymer particles comprising antibiotics which are deliverable *in situ,* as well as a method of preparation thereof. The present invention also relates to bioactive biomaterials for the controlled delivery of antibiotics comprising support materials having such polymer particles on their surface. The invention also relates to implants, prostheses, stents, lenses or cements as well as any pharmaceutical composition comprising said biomaterials.

### BACKGROUND OF THE INVENTION

The main gist of the present invention is to give the implantable devices the capacity to prevent and/or alleviate infectious processes which may follow their installation. In order to alleviate these effects it has been proposed to administer a medicament by the general route and/or to administer antibiotics locally where installation of implants occurs during bone surgery.

Since 1970 cements with antibiotics have been used in prosthetic surgery locally. In France there are 2 preparations on the market using either gentamycin or a combination of erythromycin and colimycin. It is also possible to prepare "cement with antibiotics", particularly with vancomycin, in the operating theatre in non-standard conditions. The limiting factor of this method is the uncontrolled release (in terms of concentration and duration) of the active ingredients used. Actually, the kinetics of release of the active ingredient is not controlled since no device makes it possible to adjust its delivery and therefore to perpetuate its action over a predefined duration. Moreover, part of the active ingredient may not be released because it is trapped too deep in the cement.

In order to remedy these drawbacks, systems for delivery of active ingredients, so-called "drug delivery systems (DDS)" have been developed. The principle of these drug delivery systems is to deliver pharmacologically active substances *in situ,* in a prolonged and regular manner, in a sufficient and non-toxic quantity.

In that context, stimulable polymers, namely which are polymers sensitive to an external stimulus such a variation in pH or temperature, have already been described which exhibit reactive functions obtained by encapsulation or adsorption of the active ingredients directly in the material or in beads which are themselves adsorbed or grafted on the material. However, adsorption does not allow a controlled release of the active ingredient. As regards encapsulation, when it can allow, on the one hand, a controlled release of the active ingredient, on the other hand, it proves incompatible with prolonged use and/or when the material is subjected to high stresses (flux, friction, etc.).

EP1771492 and EP1758621 patents disclose polymer particles having a reactive function, optionally engaged in a bond with an active ingredient or a biological molecule such as a protein, the said reactive function being covalently bonded to the said polymers is pH-sensitive. Such pH control presents the advantage to deliver the active ingredients only if necessary and the active ingredients kinetics are modulated by the pH decrease which typically occurs when infection rises. However, it would be useful to have polymer particles where the active ingredients comprised therein can be released in higher quantities at a specific location as to eradicate infections that can rise not only during the surgery to implant the device but also later on.

There is thus a need to have polymer particles or a biomaterial comprising the same where active ingredients comprised therein, and more specifically antibiotics, can be delivered in a tunable manner depending on the degree of infection and can potentially eradicate such infection efficiently and over a long period of time. There is also a need to provide polymer particles or a biomaterial comprising the same where at least two antibiotics can present satisfactory anti-bacteria properties for a wide range of bacteria spectra in a controlled and prolonged manner.

### SUMMARY OF THE INVENTION

In this context, the inventors made up polymer particles and biomaterials comprising the same that contain one, two or more antibiotics which can be delivered in different and controllable manners.

It is an object of the invention to provide polymer particles, the said particles being formed by polymer chains containing about 30 to 10000 monomer units, identical or different, derived from polymerization of monocyclic or polycyclic alkenes, wherein at least one of the said monomer units is substituted by a chain R comprising a polyethyleneglycol-polyglycidol chain of formula (I), wherein formula (I) is as follows:

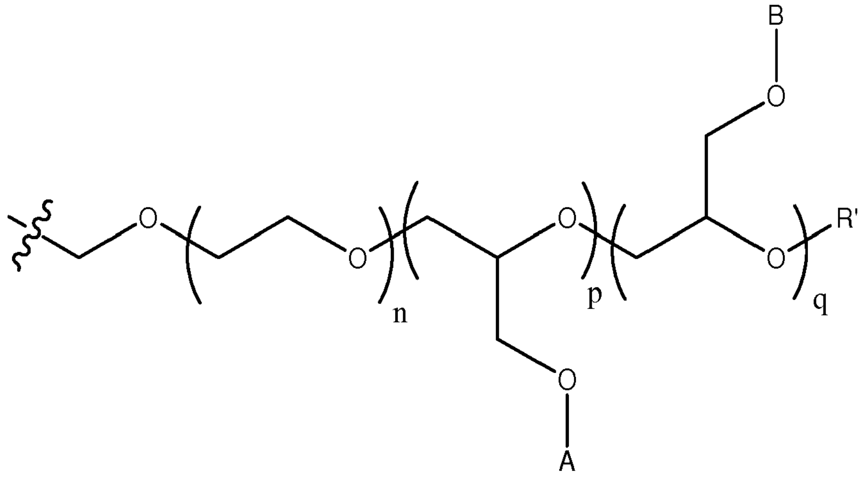

(I),

formula (I) wherein:

n represents an integer from about 0 to 300, especially from 10 to 100, p represents an integer from about 0 to 300, q represents an integer from about 0 to 300, with n+p+q is from about 10 to 300,

A represents a hydrogen atom or a group of the following formula (II):

-CONHAb1, where Ab1 represents an antibiotic with extracellular action,

B represents a hydrogen atom or a group of the following formula (III):

-CH2CNAb2, wherein Ab2 represents an antibiotic with intracellular action,

R' represents a hydrogen atom, -CH2CNAb2 or -CONHAb1 as defined above, with the proviso that when p is different from 0, then q is 0 and R' represents a hydrogen atom or -CONHAb1 as defined above, when q is different from 0, then p is 0 and R' represents a hydrogen atom or -CH2CNAb2, when p+q is not zero, at least one of the p or q moieties comprises the formula (II) or (III) respectively, and when said particles are formed by polymer chains with p+q is 0 exclusively, then at least one of said polymer chains presents a R chain comprising a polyethyleneglycol-polyglycidol chain of formula (1) where R' is -CONHAb1 as defined above,

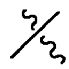

represents a covalent bond by which the polyethyleneglycol-polyglycidol chain is attached to the remainder of the R chain,

and wherein at least oneof said monomer units, identical or different from the monomer units substituted by the R chain, is substituted by a group X, wherein X represents an alkyl or alkoxy chain with about 1 to 500 carbon atoms, preferably 40 to 400 carbon atoms, comprising a reactive function of the OH, halogen, NH2, C(O)X1 type, wherein X1 represents a hydrogen atom, a halogen atom, an OR" or NHR" group, in which R" represents a hydrogen atom or an alkyl group.

The invention also relates to biomaterials comprising a support material having on its support surface covalently bonded polymer particles as defined above.

The invention also relates to a monocyclic or polycyclic alkene based macromonomer, useful as a starting material for the preparation of particles as defined above.

The invention relates more specifically to particles that have generally a spherical form and have more preferably a diameter between 50 nm and 10 μm, preferably between 300 and 400 nm.

The invention also relates to the use of biomaterials as defined above for the preparation of implantable medical devices, in particular in the form of lenses, implants, prostheses, stents or cements, in particular in ocular, vascular,

endovascular or bone surgery or treatment.

The invention also relates to medical devices, including implants, prostheses, stents, lenses or cements as well as any pharmaceutical composition, comprising biomaterials as defined above.

## BRIEF DESCRIPTION OF THE FIGURES

**Figure 1:** Size distributions of the PNb-PGLD particles measured by Dynamic light scattering (DLS) in EtOH/$CH_2Cl_2$ (65/35 %v/v) and in water

**Figure 2:** Distribution profiles of the particle size functionalized with carboxylic acid groups and Vancomycin measured by DLS in the reaction solvent (EtOH/$CH_2Cl_2$ mixture) and in DMF. For each solvent, the measure has been carried out three times (measures 1-3).

**Figure 3:** Scanning electron microscopy (SEM) observation of the titanium surface after grafting of particles functionalized with carboxylic acid groups and Vancomycin

**Figure 4:** Size distributions of polynorbornene-poly(ethylene oxide)-poly(ethylene oxide)-*bloc*-polyglycidol particles measured by DLS in the reaction medium (EtOH/$CH_2Cl_2$), in water and in DMF

**Figure 5:** Transmission electron microscopy (TEM) observations of the polynorbornene-poly(ethylene oxide)-poly(ethylene oxide)-*bloc*-polyglycidol particles

**Figure 6:** Size distributions of polynorbornene-poly(ethylene oxide)-poly(ethylene oxide)-*bloc*-polyglycidol particles functionalized with GS measured by DLS in the reaction medium (EtOH/$CH_2Cl_2$), in water and in DMF

**Figure 7:** MICs measurements were determined as the minimal concentration for which the lowest absorbance. Results are given for Vancomycin alone (Vanco), Macromonomer Vancomycin (Nb-PEO-Vanco; macro Vanco, as obtained by example 3.b)); particles grafted with Vancomycin as obtained by example 3 c) (Vanco particles); macro OH (equivalent to macro Vanco without Vancomycin), OH particles (equivalent to Vanco particles without Vancomycin)

## DETAILED DESCRIPTION

The present invention relates therefore to polymer particles and biomaterials as defined above.

The term "alkyl" as used herein is a branched or unbranched saturated hydrocarbon group of 1 to 10 carbon atoms, for example, 1 to 8 carbon atoms, or 1 to 6 carbon atoms, such as methyl, ethyl, n-propyl, isopropyl, n-butyl, isobutyl, s-butyl, t-butyl, n-pentyl, isopentyl, s-pentyl, neopentyl, hexyl, heptyl, octyl, nonyl, decyl, and the like. The alkyl group can also be substituted (by halogen atoms or alkoxy groups for instance) or unsubstituted. For example, the term "halogenated alkyl" specifically refers to an alkyl group that is substituted with one or more halide, e.g., fluorine, chlorine, bromine, or iodine. The alkyl group can also be interrupted by two or more heteroatoms, such as sulfur, nitrogen, or oxygen atoms. For example, the term "alkyl group" can specifically refer to polyoxyethylene or polyoxypropylene group.

The terms "alkoxy" and "alkoxyl" as used herein to refer to an alkyl group as defined above bonded through an ether linkage (-O-). The term "alkoxyalkyl" specifically refers to an alkyl group that is substituted with one or more alkoxy groups, as described above.

The term "halogen atom" includes chlorine, fluorine, iodine, or bromine.

As used herein, the term "about" will be understood by a person of ordinary skill in the art and will vary to some extent on the context in which it is used. If there are uses of the term which are not clear to persons of ordinary skill in the art given the context in which it is used, "about" will mean up to plus or minus 10% of the particular term.

According to a specific embodiment, the monocyclic alkene presents a number of carbon atoms constituting the ring of about 4 to 12 or the polycyclic alkene presents a number of carbon atoms constituting the rings of about 6 to 20.

The invention relates more specifically to particles or biomaterials as defined above, wherein the monomer units are derived from the polymerization of monocyclic alkenes and are of the following formula (Z1):

=[CH-R1-CH]= (Z1)

wherein R1 represents a hydrocarbon chain with 2 to 10 carbon atoms, saturated or unsaturated.

The invention relates more specifically to particles or biomaterials as defined above, wherein the monocyclic alkenes from which the monomer units are derived are:

cyclobutene leading to a polymer comprising monomer units of formula (Z1a) below:

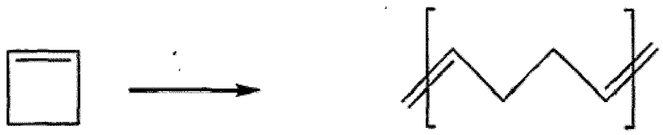

Z1a

cyclopentene leading to a polymer comprising monomer units of formula (Z1b) below:

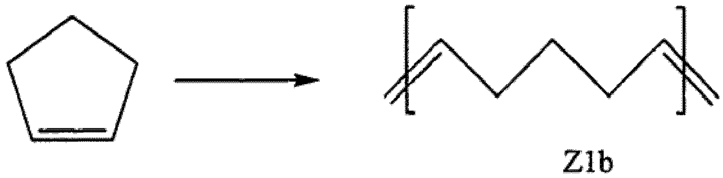

Z1b

cyclopentadiene leading to a polymer comprising monomer units of formula (Z1c) below:

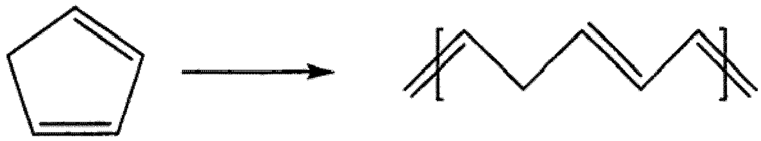

Z1c

cyclohexene leading to a polymer comprising monomer units of formula (Z1d) below:

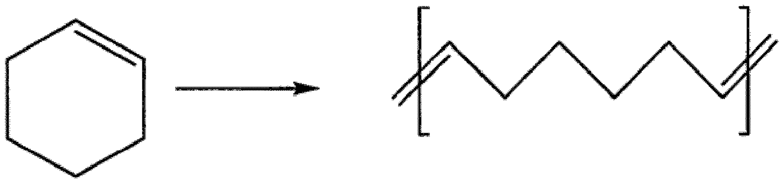

Z1d

cyclohexadiene leading to a polymer comprising monomer units of formula (Z1e) below:

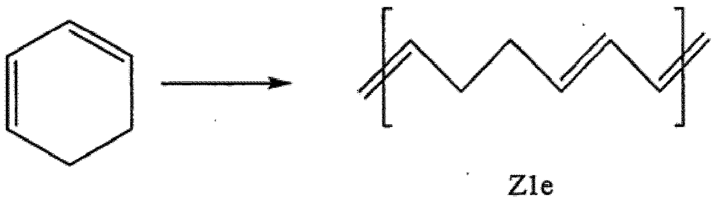

Z1e

cycloheptene leading to a polymer comprising monomer units of formula (Z1f) below:

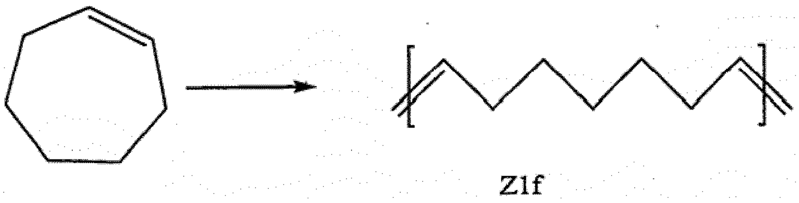

Z1f

cyclooctene leading to a polymer comprising monomer units of formula (Z1h) below:

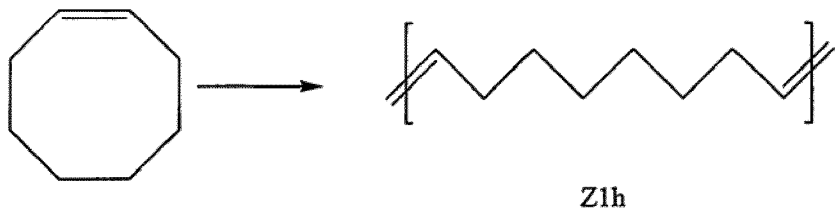

Z1h

cyclooctapolyene, especially cycloocta-1,5-diene, leading to a polymer comprising monomer units of formula (Z1i) below:

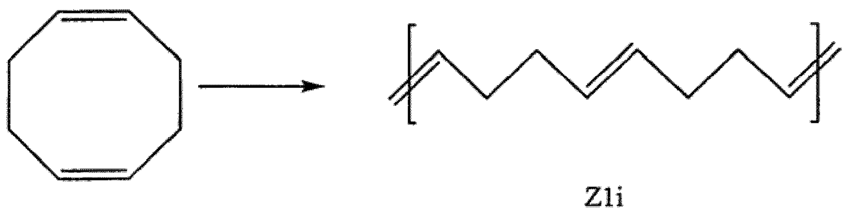

Z1i

cyclononene leading to a polymer comprising monomer units of formula (Z1j) below:

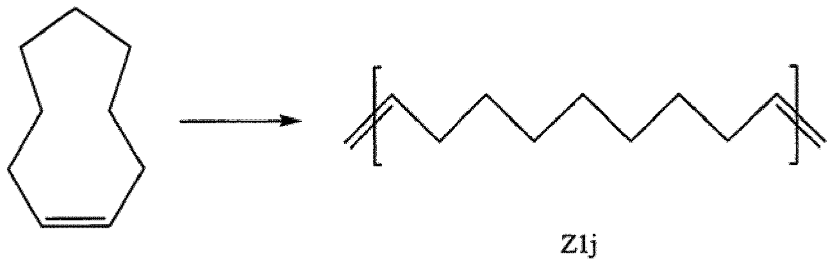

Z1j

cyclononadiene leading to a polymer comprising monomer units of formula (Z1k) below:

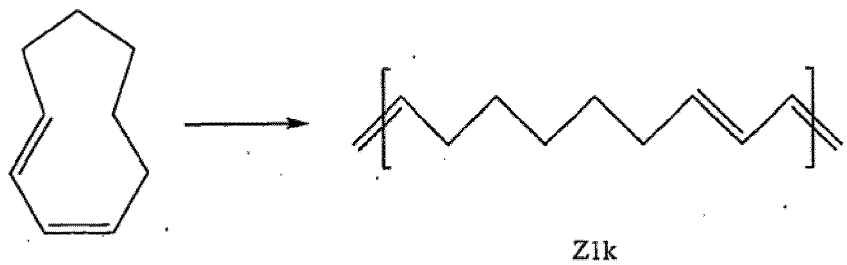

Z1k

cyclodecene leading to a polymer comprising monomer units of formula (Z1l) below:

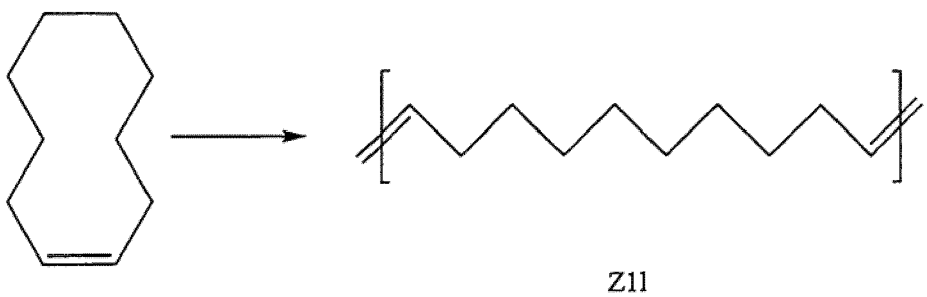

Z1l

cyclodeca-1,5-diene leading to a polymer comprising monomer units of formula (Z1m) below:

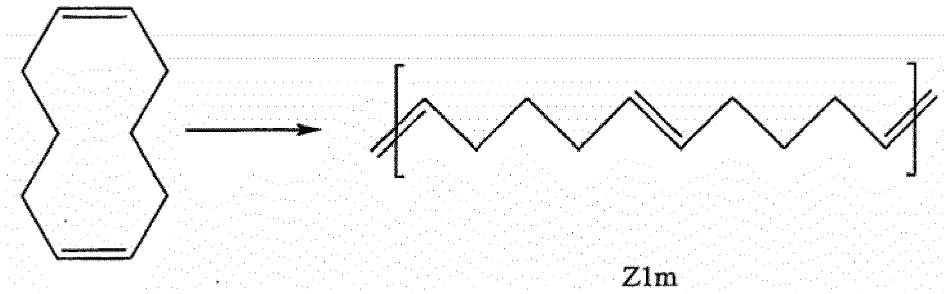

Z1m

cyclododecene leading to a polymer comprising monomer units of formula (Z1n) below:

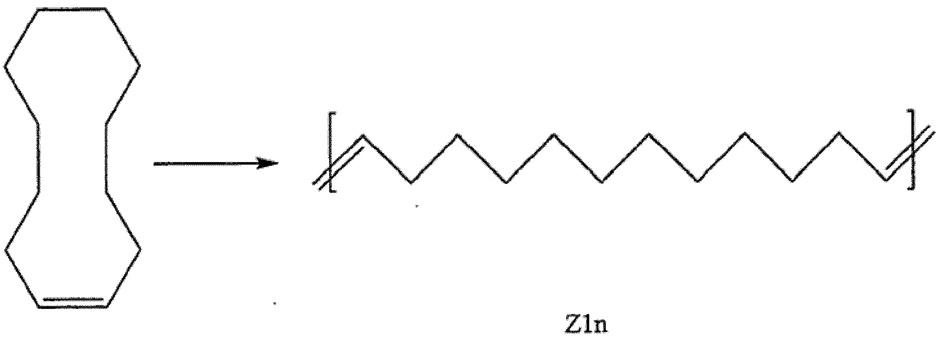

Z1n

or also 2,3,4,5-tetrahydrooxepin-2-yl acetate, cyclopentadecene, paracyclophane, ferrocenophane.

The invention also relates to particles or biomaterials as defined above, wherein the monomer units are derived from the polymerization of polycyclic alkenes and are:

- of formula (Z2) below:

  =[CH-R2-CH]= (Z2)

  wherein R2 represents:

  * a ring of formula

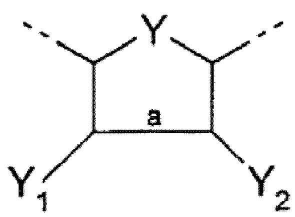

wherein:

Y represents -CH2-, or a heteroatom, or a -CHR- group , or a -CHX- group, R chain and X being as defined above,
Y1 and Y2, independently of one another, represent H, or a chain R, or a group X, as mentioned above, or form in association with the carbon atoms bearing them a ring with 4 to 8 carbon atoms, this ring being optionally substituted by a chain R or a group X as mentioned above,
a represents a single or double bond,

  * or a ring of formula

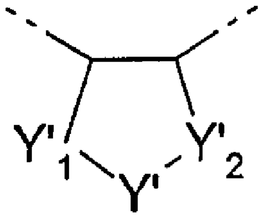

wherein:

Y' represents -CH2-, or a heteroatom, or a -CHR- group, or a -CHX- group, R and X being as defined above, Y'1 and Y'2 independently of one another represent -CH2-, or a -C(O) group, of a - COR group, or a -C-OX group, R and X being as defined above,

- of formula (Z3) below:

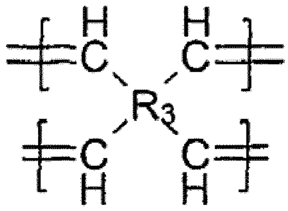

(Z3)

in which R3 represents:

* a ring of formula

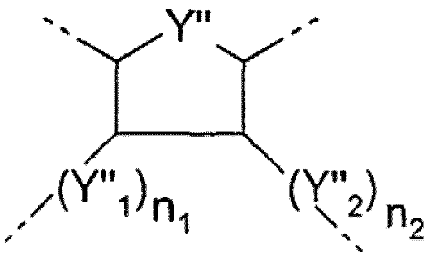

wherein:

n1 and n2, independently of one another, represent 0 or 1,
Y" represents -CH2-, or a -CHR- group, or a -CHX- group, R and X being as defined above,
Y"1 and Y"2 independently of one another represent a hydrocarbon chain with 0 to 10 carbon atoms,

*or a ring of formula

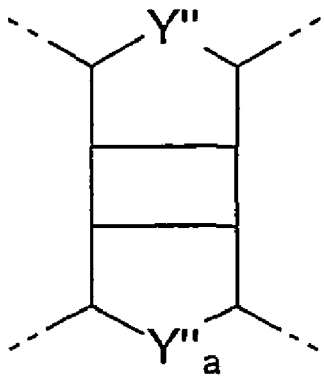

wherein Y" and Y"a independently of one another represent -CH2-, or a -CHR- group, or a -CHX- group, R and X being as defined above,

* or a ring of formula

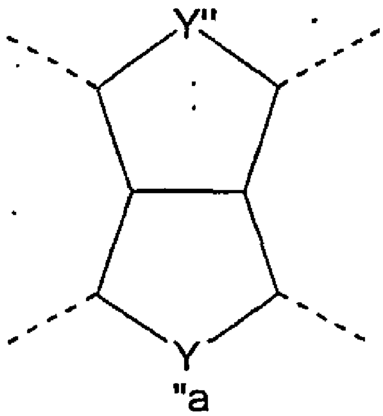

wherein Y" and Y"a independently of one another represent -CH2-, or a -CHR- group, or a -CHX- group, R and X being as defined above.

The invention relates more specifically to particles as defined above, wherein the polycyclic alkenes from which the monomer units are derived are:

- monomers containing a cyclobutene ring leading to a polymer comprising monomer units of formula (Z2a) below:

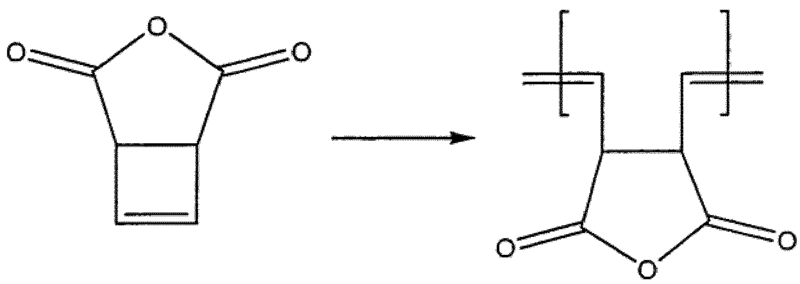

(Z2a)

- monomers containing a cyclopentene ring leading to a polymer comprising monomer units of formula (Z2b) below:

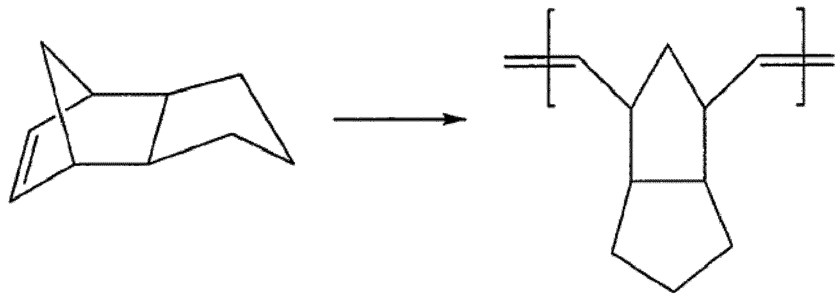

(Z2b)

- (bicyclo[2.2.1]hept-2-ene)norbornene leading to a polymer comprising monomer units of formula (Z2c) below:

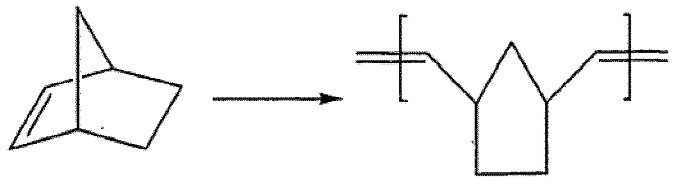

(Z2c)

- norbornadiene leading to a polymer comprising monomer units of formula (Z2d) below:

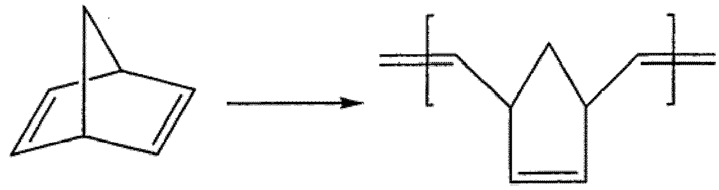

(Z2d)

- 7-oxanorbornene leading to a polymer comprising monomer units of formula (Z2e) below:

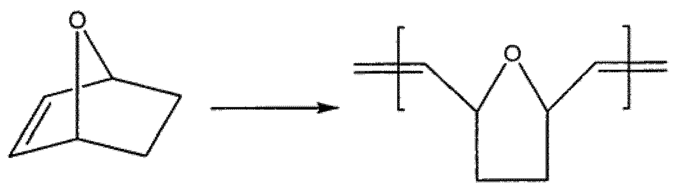

(Z2e)

- 7-oxanorbornadiene leading to a polymer comprising monomer units of formula (Z2f) below:

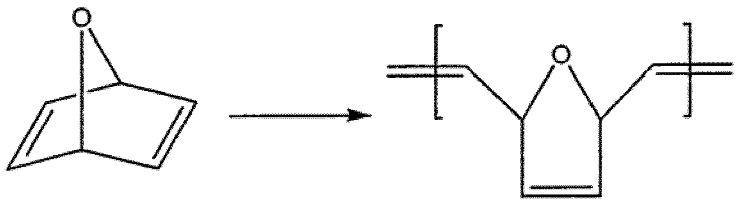

(Z2f)

- the dimer of norbornadiene leading to a polymer comprising monomer units of formula (Z3a) below:

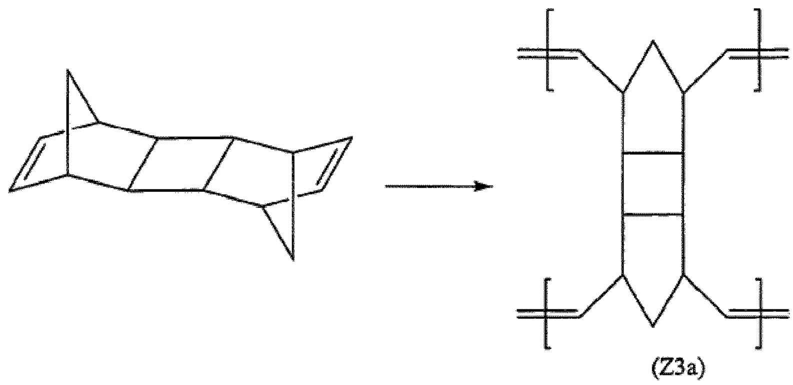

(Z3a)

- dicyclopentadiene leading to a polymer comprising monomer units of formula (Z3b) below:

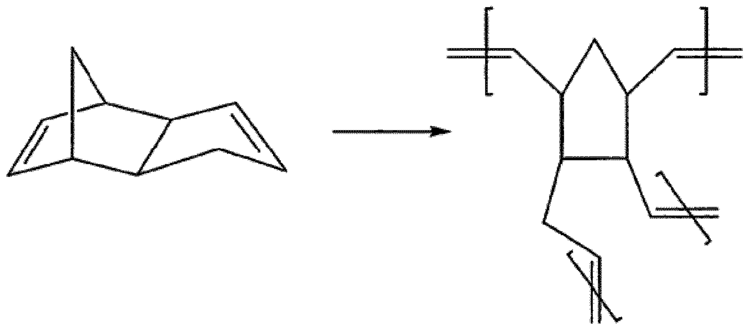

(Z3b)

- tetracyclododecadiene leading to a polymer comprising monomer units of formula (Z3c) below:

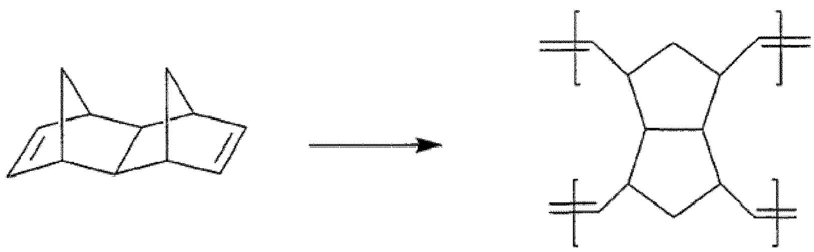

(Z3c)

or bicyclo[5.1.0]oct-2-ene, bicyclo[6.1.0]non-4-ene.

The invention relates more specifically to preferred particles or biomaterials as defined above, wherein the monocyclic or polycyclic alkenes from which the monomer units are derived are:

norbornene (bicyclo[2.2.1]hept-2-ene) leading to a polymer comprising monomer units of formula (Z2c),
tetracyclododecadiene leading to a polymer comprising monomer units of formula (Z3c),
dicyclopentadiene leading to a polymer comprising monomer units of formula (Z3b), the dimer of norbornadiene leading to a polymer comprising monomer units of formula (Z3a),
cycloocta-1,5-diene leading to a polymer comprising monomer units of formula (Z1i), preferably the monocyclic or polycyclic alkenes from which the monomer units are derived is:

norbornene (bicyclo[2.2.1]hept-2-ene) leading to a polymer comprising monomer units of formula (Z2c).

Advantageously the particles or biomaterials as defined above are characterized in that at least 0.5% up to 100% of the monomer units are substituted by a chain R as defined above, the said chain R being identical or different for these monomers.

The invention relates more specifically to particles or biomaterials as defined above, characterized in that they comprise:

between about 0.5% and 99.5% of monomer units substituted by a chain R as defined above, the said chain R being identical for these monomers, and between about 0.5% and 99.5% of monomer units substituted by a chain R as defined above, the said chain R of these monomers being different from the chain R of the preceding monomers (for instance, one chain R can comprise groups of formula (II) and the other chain R can comprise groups of formula (III)), and between 0.0% and about 99% of unsubstituted monomer units, optionally at least one of the monomer units substituted by a chain R is also substituted by a group X,

and/or between about 0.5% and 99.5% of monomer units substituted by a chain R as defined above, the said chain R being identical or different for these monomers, and between about 0.5% and 99.5% of unsubstituted monomer units, optionally at least one of the monomer units substituted by a chain R is also substituted by a group X,

and/or between about 0.5% and 99.5% of monomer units substituted by a group X as defined above, and between about 0.5% and 99.5% of monomer units substituted by a chain R as defined above, the said chain R being identical or different for these monomers, and between 0.0% and about 99.0% of unsubstituted monomer units,

the total of the percentages of the monomers mentioned above being 100%.

According to a specific embodiment, when particles of the invention are formed by polymer chains with p+q is 0 exclusively, then at least one of said polymer chains presents a R chain comprising a polyethyleneglycol-polyglycidol chain of formula (I) where R' is -CONHAb1 as defined above. This embodiment includes for instance particles where a polymer chain comprises at least one of the monomer units substituted by a chain R comprising a polyethyleneglycol-polyglycidol chain of formula (1) where p+q is 0 with R'=CH2CNAb2, then another polymer chain of said particles may comprise monomer units substituted by a chain R of formula (I) where p+q is different from 0 or monomer units substituted by a chain R of formula (I) where p+q is 0 and R' is CONHAb1.

The invention relates more specifically to particles or biomaterials as defined above, wherein the chain or chains R substituting the monomers are represented by the formula (I) as defined above, more specifically wherein at least one, or all (if compatible), of the following specific embodiments are fulfilled:

n+p+q is from 10 to 100; and/or
n is from 35 to 70, more specifically n is from 40 to 60 (e.g. n=45); and/or
either p or q is from 1 to 300.

According to the invention, the term "p moiety" represents the glycidol moiety in the parenthesis of formula (1) where p is the number of said units.

According to the invention, the term "q moiety" represents the glycidol moiety in the parenthesis of formula (1) where q is the number of said units.

According to the invention, when p+q is not zero, at least one of the p or q moieties comprises the formula (II) or (III) respectively, this embodiment includes polymer particles where Ab1 or Ab2 is present, preferably Ab2 is present, more preferably gentamicin, or both Ab1 and Ab2 are present. When both Ab1 and Ab2 are present, this entails that at least two different R chains are present in the polymer particles of the invention, ones with Ab1 and other ones with Ab2.

According to a specific embodiment, the particles or biomaterials are as defined above, wherein the chain of formula (I) is of the following formula:

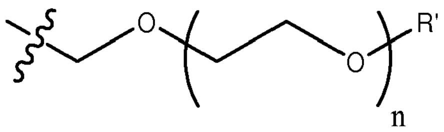
,

wherein R' is -CONHAb1 and n is as defined above, and preferably n is from 1 to 300, more preferably from 10 to 100, or Ab1 is preferably vancomycin or a salt thereof.

According to another specific embodiment, the particles or biomaterials are as defined above, wherein R' in formula (I) is an hydrogen atom.

According to another specific embodiment, the particles or biomaterials are as defined above, wherein R' in formula (I) is an hydrogen atom and q is 0.According to another specific embodiment, the particles or biomaterials are as defined above, wherein R' in formula (1) is an hydrogen atom and p is 0.

Such particles or materials are especially advantageous since they permit a controlled action of the antibiotics, e.g. vancomycin and/or gentamicin, in an effective amount to prevent and/or alleviate infectious processes which may occur for instance during surgery of the implants or later on.

Where B represents a group of the following formula (III), the particles according to the invention are stimulable particles, that is to say they are sensitive to a stimulus such as a variation in pH, which then allows the release of the antibiotics Ab2 (such as gentamycin) bonded onto these particles.

The biomaterials according to the invention as defined above are advantageously materials wherein the support material is chosen from:

- metals or oxides thereof, preferably titanium or TiO2,
- metal alloys, in particular alloys with or without shape memory such as Ni-Ti alloys, Ti-6Al-4V alloys,
- polymers, such as polyethylene terephthalate (PET), polytetrafluoroethylene (PTFE), polyvinylidine fluoride (PVDF), polyether etherketone (PEEK), polycarbonate-urethane (PCU), polyhydroxyethylmethacrylate (PHEMA), polymethylmethacrylate (PMMA), poluethylmethacrylate (PEMA), poly(4-hydroxystyrene),
- copolymers, such as the copolymer ethylene vinyl acetate (EVA), the copolymer vinylidene fluoride-hexafluoropropylene P(VDF-HFP), poly(lactic acid)-co-poly(glycolic acid) (PLA-PGA), copolymers of polymethylmethacrylate (PMMA) and poluethylmethacrylate (PEMA),
- ceramics, such as hydroxyapatites, or compounds of hydroxyapatites and tricalcium phosphate in varied proportions, in particular in the proportions 50/50.

The invention also relates to biomaterials as defined above, wherein the reactive function situated on the support material in order to ensure the covalent bond between the said material and the said particles by reacting the reactive function of these latter of the OH, halogen, NH2, C(O)X1 type, wherein X1 represents a hydrogen atom, a halogen atom, an OR" or NHR" group, wherein R" represents a hydrogen atom or an alkyl group, with a reactive function of the material in order to form a bond of the -O-C(O)-, -NH-C(O)-, -C(O)-NH-, -C(O)O- or -C(OC)2 type, or a type of bond that can be obtained by click chemistry (bioorthogonal reaction), such as via azide/cycloalkyne reaction, chloro-oxime/norbornene reaction, tetrazine/cycloctene reaction, thiol/alkene reaction, thiol/maleimide reaction, or tetrazole/alkene reaction.

The invention relates more particularly to biomaterials as defined above, wherein the reactive function of the support material is situated on an alkyl chain having about 1 to 10 carbon atoms grafted on said material, substituted or unsubstituted, and optionally comprising one or several heteroatoms, in particular O and/or Si, in said chain.

The invention relates more particularly to biomaterials as defined above, wherein:

the reactive function of the material is an $NH_2$ function situated on an aminopropyltriethoxysilane (APTES) molecule grafted on the material (M) according to the following formulae:

A)

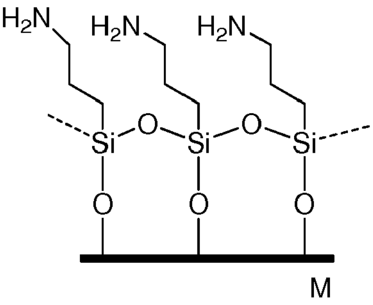

B)

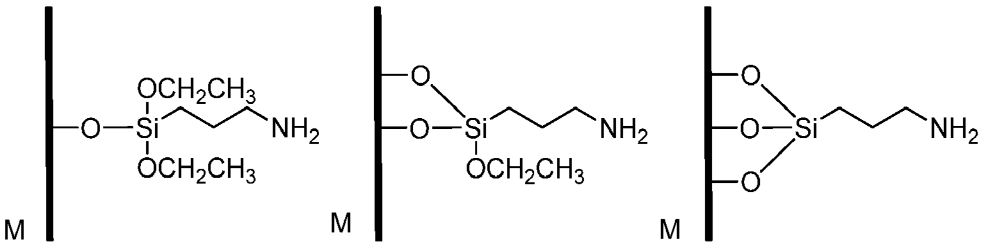

A) APTES functionalization (aqueous conditions), B) APTES functionalization (anhydrous conditions), wherein M represents a metal oxide or a ceramic such as hydroxyapatite or any other polymer having OH sites on its surface (naturally or due to prefunctionalisation),

the reactive function of the material is an $NH_2$ function situated on a surface which is coupled to COOH groups present onto particles, using for instance NHS/DCC (i.e., N-hydroxysuccinimide/Dicyclohexylcarbodiimide).

The antibiotics used in the invention are more specifically the following:

- the antibiotics with extracellular action (Ab1) are generally those that target the bacterial cell wall (such as penicillins and cephalosporins) or the cell membrane (such as polymyxins),
- the antibiotics with intracellular action (Ab2) are generally those that interfere with essential bacterial enzymes (such as rifamycins, lipiarmycins, quinolones, and sulfonamides) or those that target protein synthesis (such as macrolides, lincosamides and tetracyclines).

Among the antibiotics Ab1, one can cite the following classes: cephalosporins, including those from first to the fifth generations, such as cefalexin, cefuroxim, ceftriaxone, cefepime, ceftobiprole; carbacephem, such as Loracarbef; carbapenems, such as imipenem; glycopeptides, such as vancomycin, teicoplanin or ramoplanin; lipopeptides, such as daptomycin; monobactams, such as aztreonam; penicillins, such as amoxicillin; or polymyxins, such as polymyxin B.

According to a specific embodiment, Ab1 is a glycopeptide, preferably vancomycin or a salt thereof (such as hydrochloride).

Among the antibiotics Ab2, one can cite the following classes: aminoglycosides, including gentamicin, neomycin, and streptomycin; anzamycins, such as rifaximin; lincosamides, such as clindamycin; macrolides, such as azithromycin; nitrofuranes, such as furazolidone; oxazolidinones, such as linezolid; quinolones or fluoroquinolones, such as nalidixic acid, ofloxacin, ciprofloxacin, or levofloxacin; sulfonamides, such as sulfacetamide, furosemide; tetracyclines, such as doxycycline. According to a specific embodiment, Ab2 is an aminoglycoside, preferably gentamicin or any salt thereof (such gentamicin sulfate).

The polymer particles and biomaterials, according to a particular embodiment of the invention, comprise vancomycin and/or gentamicin, or any salt thereof (such as gentamicin sulfate)

The invention also relates to the use of biomaterials as defined above for the preparation of implantable medical devices, in particular in the form of implants, prostheses, stents, lenses or cements, in particular in vascular, endovascular or bone surgery or treatment.

The invention also relates to medical devices, more specifically implants, prostheses, stents or cements as well as any pharmaceutical composition, comprising biomaterials as defined above. It can be for instance ocular lenses, dental, ligament, valve or bone prostheses, implants, stents or cements.

The invention also relates to a pharmaceutical composition comprising particles or biomaterials as defined above, wherein said particles or biomaterials comprise antibiotics Ab1 and/or Ab2, preferably vancomycin or/and gentamicin, or any salt thereof, optionally in association with a pharmaceutically acceptable carrier, in particular for use in parenteral form.

The polymer particles, biomaterials, implants, prostheses, stents or cements as well as the pharmaceutical composition according to the invention are useful as medicines, they are more particularly for a use in the treatment of bacterial infections.

The invention also relates to a method of preparation of particles as defined above, wherein it comprises a step of polymerization of a monocyclic or polycyclic alkene as defined above substituted by a chain R as defined above, optionally in the presence of:

- one or several monocyclic or polycyclic alkenes as defined above, identical to or different from the foregoing, and substituted by a chain R as defined above, the said chain R being different from that substituting the aforementioned monocyclic or polycyclic alkene (for instance, one chain R can comprise groups of formula (II) -with antibiotic Ab1- and the other chain R can comprise groups of formula (III) -with antibiotic Ab2),
- and/or one or several monocyclic or polycyclic alkenes as defined above, identical to or different from the foregoing, and substituted by a group X as defined above,
- and/or one or several monocyclic or polycyclic alkenes as defined above, identical to or different from the foregoing, the said alkenes being unsubstituted,

the said polymerization being carried out while stirring in the presence of a transition metal complex as initiator of the reaction chosen in particular from amongst those in groups IV or VI or VII, such as ruthenium, osmium, molybdenum, tungsten, iridium, titanium, in a polar or apolar medium, particularly with the aid of the following ruthenium-based complexes: RuCl3, RuCl2(PCy3)2CHPh.

The polymerization step is preferably a ROMP reaction (Ring-opening metathesis polymerization), which can implement a wide variety of metals and range from a simple $RuCl_3$/alcohol mixture to Grubbs' catalyst.

The preparation of the particles is carried out in one step and allows the antibiotics comprised therein to be effective and/or particles having efficient kinetics of release of antibiotics depending on the envisioned uses thereof.

The invention also relates to a method of preparation of biomaterials as defined above, wherein it comprises the step as defined above, followed by a step of fixing said particles obtained in the previous step on a support material as defined above by placing the said particles in the presence of the said material, this latter having been optionally functionalized with a reactive function as defined above capable of ensuring the covalent bond between the said material and the said particles by reacting with the reactive function of the said particles.

The use of the particles makes it possible to introduce several chemical functions and antibiotics easily on the surface of the biomaterial.

Schematically, the production of the proposed device can be divided into three distinct steps:

1-The functionalization of the biomaterial
2-The synthesis of the bioactive particles (as described above)
3-The fixing of the particles on the biomaterial (as described above)

1-The Functionalization of the Biomaterial

In terms of materials, the development of a bioactive prosthesis necessitates control of the interfaces between materials and molecules or between materials and biomolecules.

Grafting is a technique which allows one or several molecules chosen for their specific properties to be fixed by covalent bonding to the surface of any type of material. The technique of functionalization can be carried out under anhydrous conditions with controlled atmosphere, temperature and pressure, which enables perfect control of the grafting conditions. In an alternative embodiment, the technique can be carried out in an aqueous solution. The technique employed comprises a modification of the functionality at the surface of the biomaterial in order to render it more reactive. Said technique is known to one skilled in the art.

The invention also relates to monocyclic or polycyclic alkenes substituted by a chain R or a group X as defined above.

The preferred monocyclic or polycyclic alkenes as defined above are chosen from amongst those mentioned above.

The invention also relates to a monocyclic or polycyclic alkene based macromonomer of formula (IV):

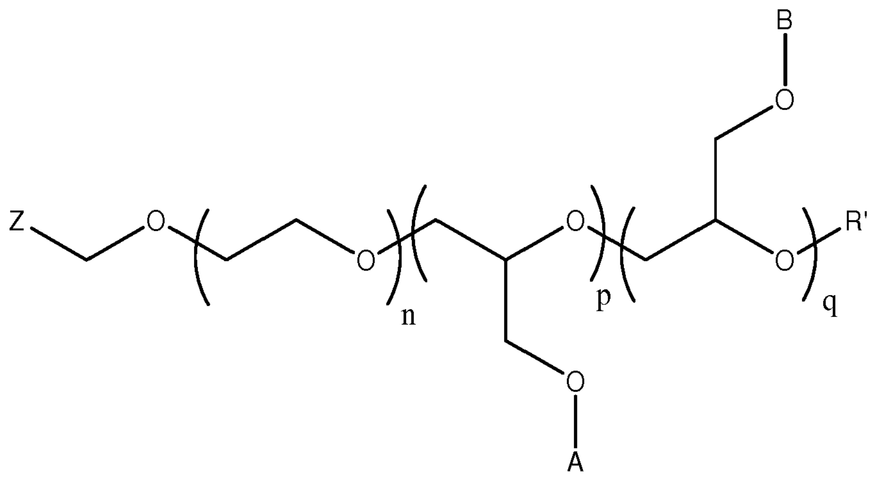

formula (IV) wherein

n represents an integer from about 0 to 300, especially from 10 to 100, p represents an integer from about 0 to 300, q represents an integer from about 0 to 300, with n+p+q is from about 10 to 300,

A represents a hydrogen atom or a group of the following formula (II):

-CONHAb1, where Ab1 represents an antibiotic with extracellular action,

B represents a hydrogen atom or a group of the following formula (III):

-CH2CNAb2, wherein Ab2 represents an antibiotic with intracellular action,

R' represents a hydrogen atom, -CH2CNAb2 or -CONHAb1 as defined above, with the proviso that when p is different from 0, then q is 0 and R' represents a hydrogen atom or -CONHAb1, when q is different from 0, then p is 0 and R' represents a hydrogen atom or -CONHAb2, when p+q is not zero, at least one of the p or q moieties comprises the formula (II) or (III) respectively, and when p+q is 0, then R' can be -CONHAb1 only,

Z represents a monocyclic or polycyclic alkene to which the polyethyleneglycol-polyglycidol chain is attached, optionally substituted by a group X, wherein X represents an alkyl or alkoxy chain with about 1 to 500 carbon atoms, preferably 40 to 400 carbon atoms, comprising a reactive function of the OH, halogen, NH2, C(O)X1 type, wherein X1 represents a hydrogen atom, a halogen atom, an OR" or NHR"group, in which R" represents a hydrogen atom or an alkyl group.

The specific or particular embodiments relative to the particles or materials described above are also included (when applicable) for the monocyclic or polycyclic alkene based macromonomers as defined by formula (IV). More specifically, Z of formula (IV) can be Z1 or Z2, as defined above.

The invention relates more particularly to monocyclic or polycyclic alkenes based macromonomers as defined above, characterized by the following formula (V):

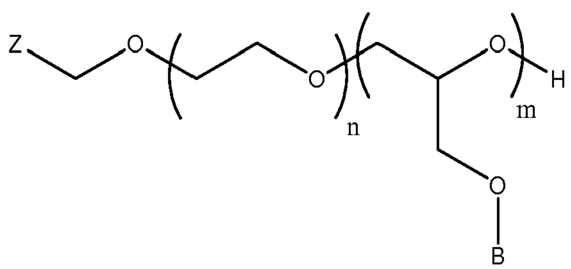

in which Z is as described above, n is as defined above, more preferably is 0, and m is q as defined above, and B is as defined above (including specific and particular embodiments), wherein at least one of the m moieties comprises the formula (III).

In a particular embodiment, the cyclic alkenes are selected from norbornene, tetracyclododecadiene, dicyclopentadiene, the dimer of norbornadiene, and cycloocta-1,5-diene. In a specific embodiment, the cyclic alkene is

norbornene, as defined above.

The invention also relates to the use of monocyclic or polycyclic alkenes based macromonomer as defined above for carrying out a method of preparation of particles or biomaterials defined above, especially by the methods described above.

The invention further relates to particles or biomaterials as defined above wherein the monocyclic or polycyclic alkenes based macromonomers are as defined above specifically.

The invention will now be illustrated by the following examples. They are not intended to be limiting. The percentages are expressed by weight, unless otherwise specified.

## EXAMPLES

### 1. Material and methods

**Material:** Ethylene oxide (EO; 99.5%; Aldrich) was stirred over sodium at -30°C for 2 hours and subsequently cryodistilled. Tetrahydrofuran (THF; J.T. Baker) was cryodistilled from sodium benzophenone before use. Ethanol (96%; purissimum grade pur; Xilab) and dichloromethane (purissimum grade pur, Xilab) were degassed before use. Diphenyl methyl potassium (DPMK; 0.64 mol.$L^{-1}$ in THF) was synthesized and dosed according to well-established procedures. Sodium hydride (60% in dispersion in mineral oil; Aldrich) was washed with anhydrous heptane before use. Grubbs first generation complex Ch-$(PCy_3)_2$Ru=CH-Ph (Aldrich; stored in a glovebox under Argon atmosphere) was used as received. Norbornene (Nb) (99% (GC); Aldrich), 5-norbornene-2-methanol (98%; mixture of endo and exo; Aldrich), bromoacetaldehyde diethyl acetal (97%; Aldrich), 2-bromoethyl acetate (97%; Aldrich), Gentamicin Sulphate (GS; C1, C1a, C2 mixture; Aldrich), were used without further purification. Titanium discs (Ti90Al6V4; Ø=5 mm; h=3 mm; Ra=5-6 μm) were purchased from Good Fellow, France. Anhydrous hexane (99%), anhydrous N-N-dimethylformamide (DMF; 99.8%), dicyclohexylcarbodiimide (DCC; 99%), 3-aminopropyltriethoxysilane (APTES; ABCR; 97%) was obtained from ABCR, France. N-hydroxysuccinimide (NHS; 98%) was purchased from Alfa Aesar, France. Vancomycin hydrochloride was purchased from Aldrich. 4-Dimethylaminopyridine (DMAP, 99%) and disuccinimidyl carbonate (DSC; 98%) were obtained from Acros, France.

**Methods:** ROMP (Ring-Opening Metathesis Polymerization) was performed in a glovebox. $^1H$ NMR spectra were obtained using a Bruker spectrometer 400 MHz in $CDCl_3$, $D_2O$ or DMSO-$d_6$ used as solvent. Size exclusion chromatography analyses were carried out on a Varian apparatus equipped with TOSOHAAS TSK gel columns and a refractive index detector. THF or DMF were used as solvents at a flow rate of 1 mL.$min^{-1}$. Mass calibration was achieved with narrow polydispersity polystyrene standards. For ROMP in dispersion, conversions of Nb were determined by gas chromatography with a trace of dodecane as internal standard, using a VARIAN GC3900 (GC retention times: $t^{GC}_{Nb}$ = 1.77 min; $t^{GC}_{dodecane}$ = 8.55 min). The PEO-based macromonomer conversions were followed by SEC (SEC retention times: $t^{SEC}_{macromonomers}$ = 18.75 min;.$t^{SEC}_{dodecane}$ = 31.70 min), while the PGLD-based macromonomer conversions were determined by elemental analysis after the particle dispersion purification by ultrafiltration and lyophilisation of the particles and also by gravimetry after ultracentrifugation (Eppendorf centrifuge 5804R ; 8000 rpm ; 5 min ; 10°C) and drying under vacuum. Dynamic light scattering (DLS) measurements were performed using a MALVERN zetasizer Nano ZS equipped with He-Ne laser (4 mW; 633 nm). Before measurements, latexes were diluted about 800 times to minimize multiple scatterings caused by high concentration. The scattering angle used was 173°. TEM pictures were performed with a HITACHI H7650 microscope operating at an accelerating voltage of 80 kV. For the particles size, distribution and morphology observation, samples diluted about 800 times were deposited on a 200 mesh carbon film-coated copper grids surface. The particle grafting density was characterized by SEM observations using a HITACHI S-2500 scanning electron microscope.

### 2. Synthesis of Gentamicin-functionalized particles with polyglycidol macromonomer

#### a) Synthesis of glycidol acetal:

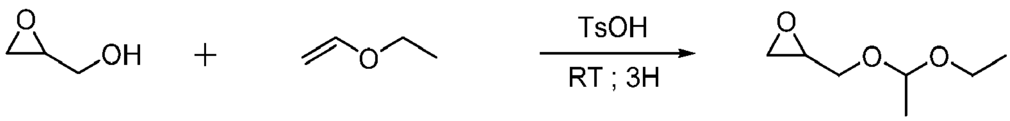


Tosylic acid (TsOH, 1 g) was added portion-wise to a magnetically stirred solution of 40.0 g of 2,3-epoxypropanol (glycidol) in 200 mL of ethyl vinyl ether. The temperature was maintained below 40°C with an ice-water bath. The reaction

mixture was stirred for 3 hours. Then, the reaction mixture was washed with 100 mL of $NaHCO_3$ saturated water solution, the organic phase was dried with $MgSO_4$, filtrated, and the solvent (ethyl vinyl ether) was evaporated under reduced pressure. The product was purified by distillation under reduced pressure.
Yield: 71%
$^1H$ NMR data in $CDCl_3$: δ (ppm) 0.8-1.2 (m ; 6H ; $-CH_3$) ; 2.4-2.7 (m ; 2H $CH_2$ acetal) ; 3.05 (m ; 1H ; CH glycidol) ; 3.3-3.9 (m ; 4H ; $CH_2$ glycidol) ; 4.85 (t; 1H ; CH acetal)

**b) Synthesis of α-norbornenyl-poly(glycidol acetal)macromonomer:**

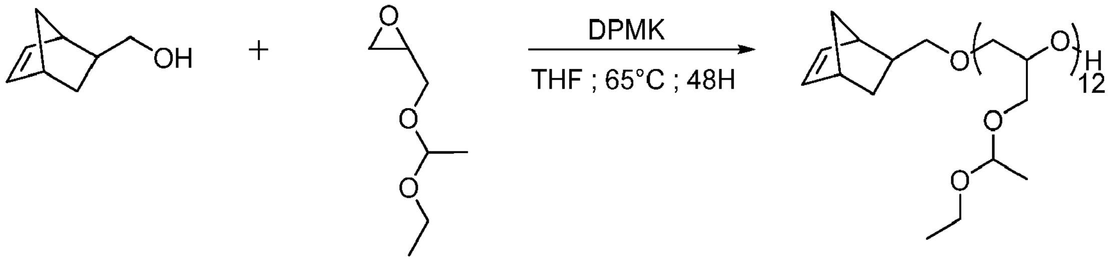
DPMK
THF ; 65°C ; 48H

For a macromonomer with a number-average polymerization degree ($DP_n$) of 12, 0.44 mL of the initiator 5-norbornene-2-methanol was added to 200 mL of freshly cryodistilled THF, following by the addition of 4.5 mL of DPMK solution (0.64 $mol.L^{-1}$ in THF). Then, Glycidol acetal, (6.55 mL) was added to the reaction medium. For a full conversion, the reaction was left stirring for approximately 24 hours at 65°C. 5-7 drops of degassed acidified methanol were added to the reaction mixture to desactivate the polymerization. After a 2-3 days dialysis in ethanol, the solution was filtered and the solvent was removed by rotary evaporation and the macromonomer was dried under vacuum.
Yield: 67%
$^1H$ NMR data in $CDCl_3$: δ (ppm) (400 MHz): 1.12-1.95 (80H, $-CH_3$); 2.35-2.45 (3H, - $CH-_{cycle}$); 3.49-3.99 (96H, -O-CH-$CH_2$-O- and CH-$CH_2$-OAc); 4.71 (13H ; CH) 6.00-6.28 (2H ; $-CH=CH-_{cycle}$)

**Characteristics of the α-norbornenyl-poly(glycidol acetal)macromonomers**

| $DP_{n;Th}$[1] | $DP_{n;NMR}$[2] | $M_{n;NMR}$ ($g.mol^{-1}$)[3] | $M_{n;SEC}$ ($g.mol^{-1}$)[4] | PDI[5] |
|---|---|---|---|---|
| 13 | 12 | 1910 | 1630 | 1.06 |
| 27 | 25 | 3830 | 3710 | 1.06 |
| 54 | 50 | 7440 | 7630 | 1.09 |

[1] $DP_{n;Th}=n_{mono}/n_{NbOH}$ with $n_{mono}$ the initial amount of monomer and $n_{NbOH}$ the initial amount of norbornene methanol.
[2] $DP_{n;NMR}=2I_{CH}/I_{Nb}$ with $I_{Nb}$: integration of the ethylenic protons of the norbornenyl entity, $I_{CH}$: integration of the CH proton of the acetal entity
[3] $M_{n;NMR}=M_{Nb}+146DP_{n;NMR}$ $M_{Nb}$ molecular weight of the norbornenyl entity, 146: molecular weight of the glycidol acetal unit.
[4] molecular weight measured by Size Exclusion Chromatography
[5] PDI: Polydispersity index

**c) Synthesis of α-norbornenyl-polyglycidol macromonomer:**

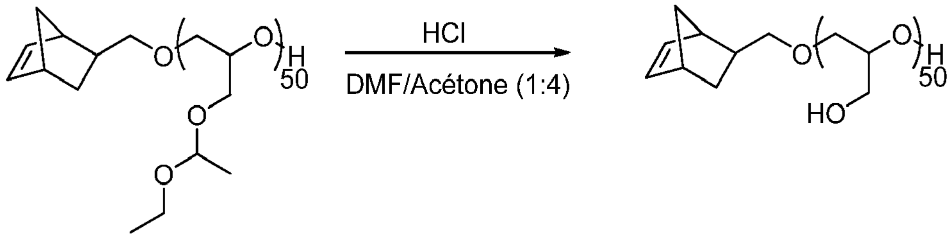


The α-norbornenyl-poly(glycidol acetal) macromonomer (1.65 g ; $DP_n$=50 ; $M_n$=7440 g/mol) was dissolved in 75 mL of a mixture of N,N-dimethylformamide (DMF)/acetone (1:4 v/v), and 4.5 mL of concentrated hydrochloric acid (HCl) solution (11.7 mol.$L^{-1}$) were added. The reaction was stirred for 1 hour, and then a saturated $Na_2CO_3$ aqueous solution was added to neutralize HCl until pH=8 (monitored with pH paper). The solvent was evaporated under reduced pressure and the macromonomer was redissolved in 50 mL of ethanol and filtrated to remove residual salts. Then, ethanol was evaporated, and the macromonomer was redissolved in 50 mL of water. Purification of the sample was carried out by a 2-3 days dialysis in water, then the macromonomer was lyophilized.

- Yield: 85%
- $^1H$ NMR data in $D_2O$: δ (ppm) (400 MHz): 1.12-1.95 (m, 4H, $-CH_2$-cycle); 2.35-2.45 (m, 3H, $-CH-_{cycle}$); 3.49-3.99 (2m, 125H, $-O-CH-CH_2-O-$ and $CH-CH_2-OH$); 6.00-6.28 ($-CH=CH-_{cycle}$)
  $DP_{n;NMR}$=53; $M_{n:NMR}$=3900 g/mol
- SEC in DMF: $M_n$=4000 g.$mol^{-1}$; PDI=1.15

**d) Partial deprotection of α-norbornenyl-poly(glycidol acetal) macromonomer**

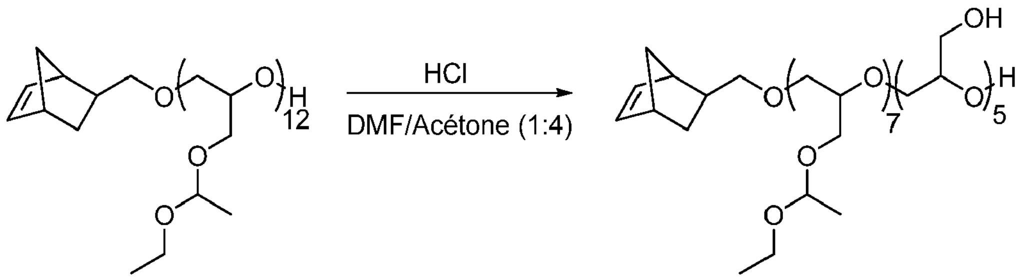


α-norbornene-poly(glycidol acetal) macromonomer (1.65 g) was added to a 75 mL solution of N,N-dimethylformamide (DMF)/acetone (1:4 v/v). Then, 0.64 mL of concentrated hydrochloric acid (HCl) solution (11.7 mol/L) were added. The reaction was stirred for 4 min, then a saturated $Na_2CO_3$ aqueous solution was added to neutralize the HCl until pH=8 (monitored with pH paper). The solvent was evaporated and the product was dissolved in ethanol. The residual salts were removed by filtration. After a 2-3 days dialysis, the product was lyophilized.
Yield: 72% for $DP_n$=12 (1); 89% for $DP_n$=25 (2)
SEC in THF: (1): $M_n$=1270 g.$mol^{-1}$ ; PDI=1.08. (1): $M_n$=2570 g.$mol^{-1}$ ; PDI=1.08

**e) Acetal functionalization of partially deprotected macromonomers**

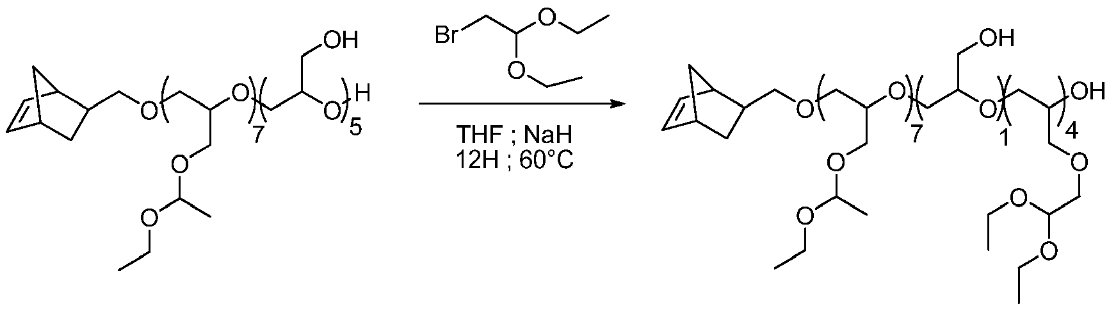


In a typical experiment, 1 g of partially deprotected α-norbornenyl-poly(glycidol acetal) macromonomer was dissolved in 30 mL of freshly cryodistilled THF. 10 equivalents of NaH (M=24 g/mol; dispersed in mineral oil 60% (w/w)), previously washed with heptane to remove the mineral oil, were dispersed in 10 mL of THF. The macromonomer solution was added dropwise to NaH under stirring and under a nitrogen flux. After 30 min, 4.5 equivalents of bromoacetaldehyde diethyl acetal (M=197 $g.mol^{-1}$; d=1.31) were added dropwise. The reaction mixture was stirred for 12 hours at 60°C. NaH was then neutralized with a 1 N HCl solution and the solution mixture was evaporated, redissolved in $CH_2Cl_2$, dried with $MgSO_4$ and filtrated. Finally, $CH_2Cl_2$ was evaporated and the product was dried under vacuum.
Yield: 72% for $DP_n$=12 (1); 89% for $DP_n$=25 (2)
SEC in THF: (1): $M_n$=1270 $g.mol^{-1}$ ; PDI=1.08. (1): $M_n$=2570 $g.mol^{-1}$ ; PDI=1.08

**f) Synthesis of GS-functionalized α-norbornenyl-polyglycidol macromonomer**

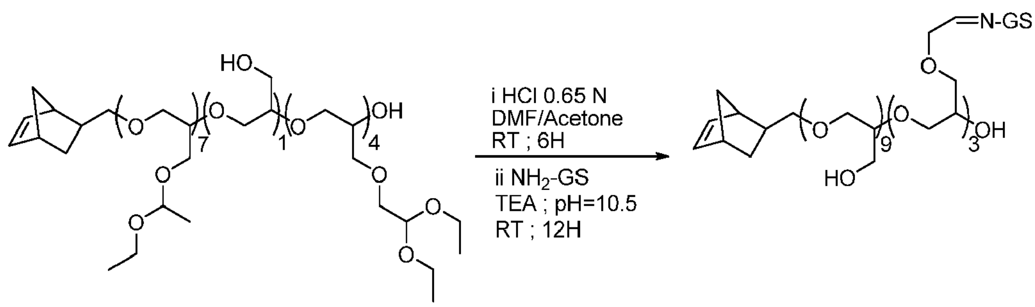


Acetal functionalized polyglycidol macromonomer (0.4 g) was dissolved in 18 mL of DMF/Acetone (1:4 v/v) and 1.1 mL of concentrated HCl (11.7 N) was added dropwise. The mixture was stirring for 6 hours at room temperature. Then the solution was basified by adding dropwise a solution of triethylamine (TEA) (1 M) dissolved in the solvent medium under nitrogen flux (pH=10). Finally, GS (5 eq.) dissolved in 10 mL of buffer solution pH=12 was added dropwise. After 12 hours, the solvent was evaporated and the product was purified by a 3-days dialysis in a TEA solution 10 mM (pH=10.5). $M_n$;NMR=2710 g/mol for $DP_n$=12 (1) and $M_{n;NMR}$=5890 g/mol for $DP_n$=25 (2).
- SEC in DMF: (1): $M_n$=1980 $g.mol^{-1}$; PDI=1.27. (2): $M_n$=3420 $g.mol^{-1}$ ; PDI=1.32

**g) Synthesis of unfunctionalized polynorbornene-polyglycidol particles**

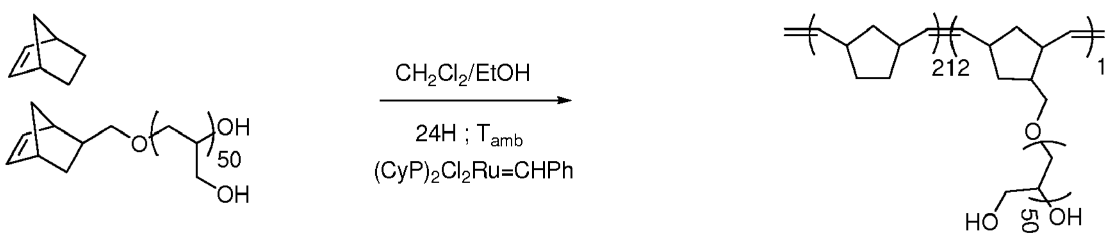


Dispersion polymerizations were carried out at room temperature under inert atmosphere (glovebox) and under

stirring. Solvents were degassed according to the freeze-pump-thaw procedure. In a typical experiment, 10 mg (3.6 $10^{-5}$ mol) of Grubbs 1st generation complex were dissolved in 3.3 mL of dichloromethane/ethanol mixture (1:1 v/v). Both norbornene (201 mg; 6.18 $10^{-3}$ mol) and α-norbornenyl-polyglycidol macromonomer (241 mg; 3.24 $10^{-5}$ mol) were first dissolved in 6 mL of dichlomethane/ethanol solution (35:65 v/v) and added to the catalyst. 206 mg of dodecane is also added as internal standard. The mixture was stirred during 24 hours. The desactivation of the reaction medium was performed by addition of 0.3 mL of ethyl vinyl ether.

- Nb conversion by GC: $\pi_{Nb}$>99%.
- Macromonomer conversion by gravimetric analysis: first 5 mL of dispersion was ultra-centrifuged (8000 rpm; 10°C; for 5 min), then the solid phase ($m^f_{sol}$=198.9 mg; PNb-PGLD) and the liquid phase ($m^f_{liq}$=144.4mg; dodecane, desactivated Grubbs I catalyst and unreacted PGLD) were dried under vacuum. These weights were compared with the theoretical weights determined by considering the introduced products before the reaction (norbornene and PGLD; $m^{th}_{sol}$=229 mg; $m^{th}_{liq}$=112 mg). The macromonomer conversion can be calculated with the following equation:

$$\pi_{PGLD} = \frac{m^f_{sol} - m^i_{Nb}}{m^{th}_{sol} - m^i_{Nb}} = 1 - \frac{m^f_{liq} - m^{th}_{liq}}{m^i_{PGLD}} = 75\%$$

$\pi_{PGLD}$ : macromonomer conversion
$m^i_{Nb}$ : initial weight of the Nb monomer
$m^i_{PGLD}$ : initial weight of the PGLD macromonomer

- Macromonomer conversion by elemental analysis: first, the particles were transferred in water and purify by ultrafiltration, then the dispersion was lyophilized. Elemental analysis: Measured: (mol%) C: 33.19; H: 58.03; O: 8.78. Theoretical values for a total conversion: (mol. %) C: 34.18; H: 56.57; O: 9.25.
  The macromonomer conversion can be calculated by considering the elemental analysis and the polymer formula $(C_7H_{10})_n$-$(C_8H_{12}O(C_3H_6O_2)_{DPn})_m$, the initial proportions and a total conversion of Nb: $\pi_{PGLD}$=94%.
- particle size measurement by DLS are presented on Figure 1:

**h) Synthesis of GS-functionalized polynorbornene-polyglycidol particles**

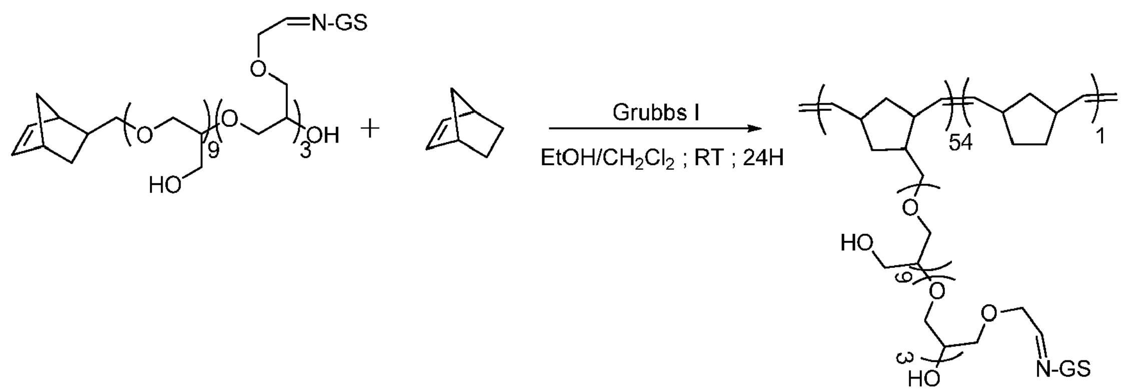


10 mg (3.6 $10^{-5}$ mol) of Grubbs 1st generation complex were dissolved in 3.3 mL of dichloromethane/ethanol mixture (1:1 v/v). Both norbornene (201 mg; 2.14 $10^{-3}$ mol) and GS-functionalized α-norbornenyl polyglycidol macromonomer (241 mg; $M_n$=3420 g/mol; 7.05 $10^{-5}$ g/mol) were first dissolved in 6 mL of dichlomethane/ethanol solution (35:65 v/v) and added to the catalyst. 206 mg of dodecane is also added as internal standard. The mixture was stirred during 24 hours. The desactivation of the reaction medium was performed by addition of 0.3 mL of ethyl vinyl ether.

- Nb conversion by GC: $\pi_{Nb}$=80%
- Macromonomer conversion by gravimetric analysis: Macromonomer conversion $\pi_{PGLD}$ was determined thanks to a gravimetric analysis. After ultracentrifugation the solid phase ($m^f_{sol}$=197.7 mg; PNb-PGLD was dried under vacuum. This weight was compared with the theoretical weight determined by considering the introduced products before

the reaction (Nb; macromonomer) and a Nb conversion of 80% ($m^{th}_{sol}$=324.8 mg). The calculated macromonomer conversion is 45%.

$$\pi_{PGLD} = \frac{m^{f}_{sol} - 0.8 m^{i}_{Nb}}{m^{th}_{sol} - 0.8 m^{i}_{Nb}} = 45\%$$

- Macromonomer conversion by elemental analysis: first, the particles were transferred in water and purify by ultrafiltration, then the dispersion was lyophilized. Elemental analysis: Measured: (mol%): C: 30.51 ; H: 63.68 ; N: 0.63 ; O: 5.18. Theoretical values for a total conversion (mol%): C: 34.06 ; H: 57.22 ; N: 2.28; O: 6.44.

Elemental analysis measured values were compared with the theoretical ones calculated from the initial state and considering a conversion of 80% for Nb and a total conversion for the macromonomer. A macromonomer conversion of 41% was determined, close to the macromonomer conversion calculated by gravimetric analysis.

- SEC in THF: $M_n$=97300 g/mol (styrene eq) PDI=1.59
- Size measurement: The DLS analysis of the dispersion showed the presence of big objects with diameters of about 5-6 μm in the ethanol/dichloromethane solvent mixture and with diameters of 2-3 μm in water after their transfer and an ultrafiltration purification step. The GS loading is about 20 $10^6$ molecules per particle versus 3 $10^6$ molecules per particle with the PEO based particles.

**3. Grafting of vancomycin-functionalized poly(ethylene oxide)-polynorbornene particles onto titanium surface**

**a) Synthesis of α-norbornenyl-ω-Succinimidyl poly(ethylene oxide)**

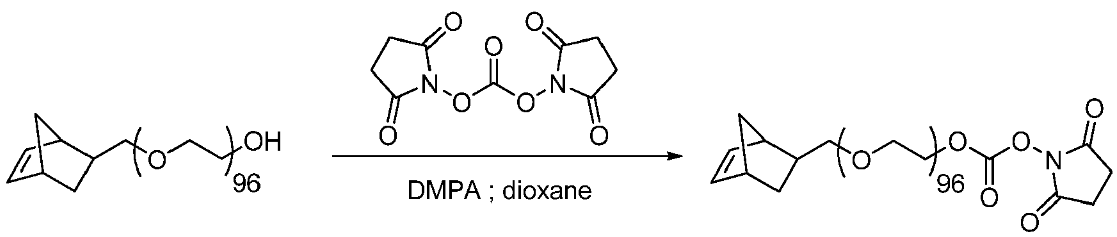


In a typical experiment, 5g of a-norbornenyl-poly(ethylene oxide) macromonomer ($M_n$=4300 g/mol; 1.16 mmol) was dissolved in 25 mL of dry dioxane and then DSC (9 mmol in 20 mL of dry acetone) was added. DMAP (9 mmol in 15 mL of dry acetone) was added slowly under magnetic stirring and the reaction was carried out at room temperature for 6 h. Nb-PEO-SC was directly precipitated from the reaction mixture by diethyl ether and then several cycles of redissolving of the product in acetone and precipitation in diethyl ether were carried out in order to remove excess DSC and DMAP. The activated product was stored dry in a glovebox.
Yield: 75%
$^1$H NMR ($CDCl_3$, 400 MHz): δ (ppm) Norbornenyl moiety, 5.85-6.04, 3.30, 3.11, 3.00, 2.84, 2.68-2.72, 2.25, 1.74, 1.62, 1.04-1.40, 0.41; EO moiety, 4.40, 3.40-3.80; Succinimidyl moiety, 2.77
$M_{n;NMR}$=4300 g/mol
functionalization: F>99%

- SEC in THF: $M_{n;SEC}$=3900 g/mol (styrene eq) PDI=1.08

**b) Synthesis of α-norbornenyl-ω-vancomycin poly(ethylene oxide)**

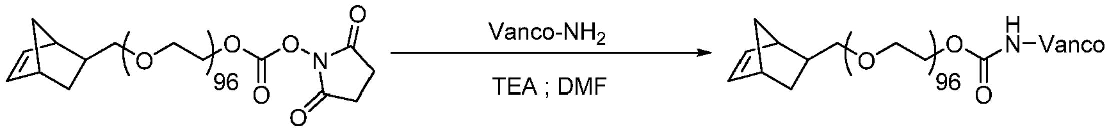


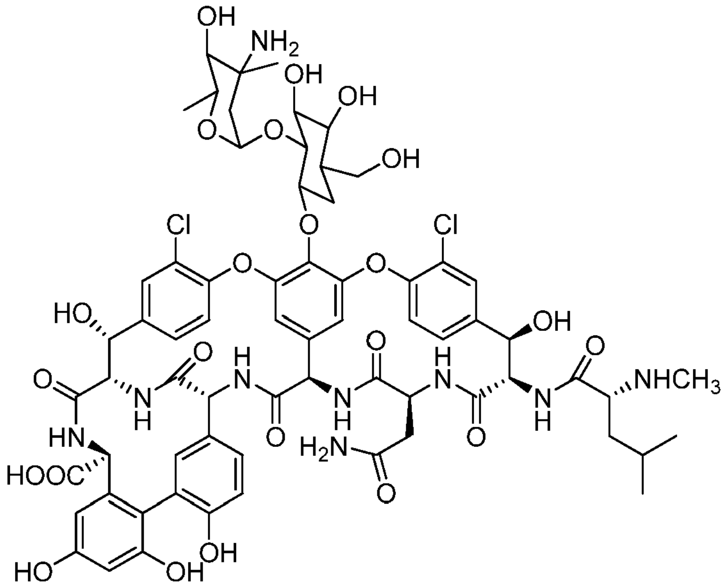

Structure of Vancomycin

To a solution of vancomycin (0.2235 g, 0.15 mmol) and triethylamine (TEA, 0.4 mL, 3.0 mmol) in anhydrous dimethylformamide (DMF, 10 mL) was added a solution of Nb-PEO-SC (0.3 g, 0.075 mmol) in anhydrous DMF (10 mL) and 2.235 g molecular sieves (4 Å). The reaction mixture was stirred at 30 °C for 12 hrs, filtered through celite, and the solid product was obtained by precipitation with diethyl ether, filtered, and dried. The product was purified by ultrafiltration using deionized $H_2O$ as the solvent and a regenerated cellulose membrane (5 K Daltons) to separate the product from unreacted vancomycin. The retained fraction was frozen with liquid nitrogen and lyophilized for 48 hrs.

- Yield: 88%
  $^1H$ NMR (DMSO-$d_6$, 400 MHz): δ (ppm) Norbornenyl moiety, 5.85-6.04, 3.30, 3.11, 3.00, 2.84, 2.68-2.72, 2.25, 1.74, 1.62, 1.04-1.40, 0.41; EO moiety, 3.40-3.80; Vancomycin moiety, 6.44-7.66, 5.29-5.49, 4.06-4.47, 3.41-3.75 (overlapped by EO moiety peak), 2.73, 1.11-2.02, 0.84.
  $M_{n;NMR}$=5100 g/mol
  functionalization: F>99%
- SEC in DMF: $M_n$=8000 g/mol (styrene eq.) PDI=1.13

**c) Synthesis of vancomycin-carboxylic acid particles**

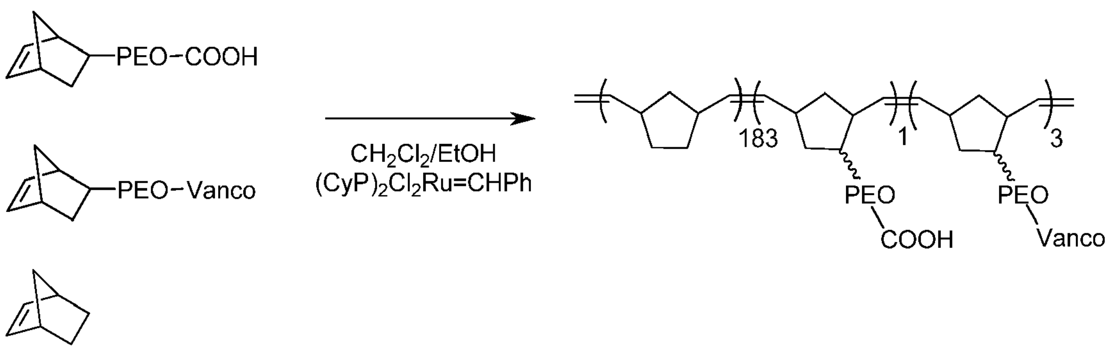


Functionalized particles were formed by ROMP in dispersion. Dispersion polymerizations were carried out at

room temperature under inert atmosphere (glovebox) and stirring. Solvents were degassed according to the freeze-pump-thaw procedure. In a typical synthesis, 30 mg ($3.6\ 10^{-5}$ mol) of Grubbs 1st generation complex were dissolved in 10 mL of dichloromethane/ethanol mixture (50/50% vs volume). Norbornene ($6.1\ 10^{-3}$ mol), α-norbornenyl-ω-carboxylic acid-poly(ethylene oxide) macromonomer ($3.7\ 10^{-5}$ mol) and α-norbornenyl-ω-vancomycin-poly(ethylene oxide) macromonomer ($1.1\ 10^{-4}$ mol) were first dissolved in 18 mL of dichlomethane/ethanol solution (35/65% V/V) and added to the Grubbs 1 solution. The mixture was stirred during 24 hours. At the end of polymerization Ruthenium end-capped chains were deactivated by addition of 0.3 mL of ethyl vinyl ether. Then, the particles were transferred to DMF to carry out the grafting step onto titanium surfaces: first DMF was added drop wise, then dichloromethane and ethanol were evaporated under reduced pressure.

- Norbornene conversion: >99%
- Global macromonomer conversion: 90%
- Distribution profiles of the particle size functionalized with carboxylic acid groups and Vancomycin : measurement by DLS given in Figure 2

**d) Particle grafting onto titanium surfaces**

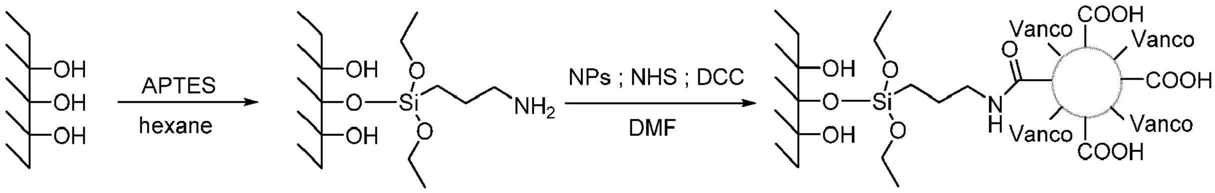


The particle grafting step was a two-step process: first, titanium surfaces were functionalized with anime groups using APTES through a well established protocol: Briefly, titanium samples were first outgassed at 150°C under vacuum ($10^{-5}$ Torr) for 20 h. Silanization of the surface was performed by immersing the substrate in a solution of APTES ($10^{-2}$ M) in anhydrous hexane under inert atmosphere (glovebox) during 2 h. Samples were washed in glovebox by two rinsings under stirring and sonication for 30 min (both steps have been performed using anhydrous hexane). Finally, samples were outgassed at 100°C under vacuum ($10^{-5}$ Torr) for 4 h. Next, the particles were covalently linked onto the titanium surface through the formation of an amide bond between the carboxylic acid groups of the particles and the amine groups present onto the surfaces (activated by NHS and DCC). In inert atmosphere (glovebox), DCC (237 mg, 82 eq.; $1.1\ 10^{-3}$ mol) and NHS (100 mg, 62 eq.; $8.7\ 10^{-4}$ mol) were diluted in 2 mL of particle dispersed in DMF ($n_{-COOH}$=$1.5\ 10^{-5}$ mol). Finally, the mixture was deposited on titanium materials and stirred for 72 h at room temperature. The samples were then washed in three successive ethanol baths, dried and stored under inert atmosphere. The grafting step was carried out three times. Between two successive steps, the materials were rinsed in ethanol baths.

SEM observation of the titanium surface after grafting of particles functionalized with carboxylic acid groups and Vancomycin is presented on Figure 3.

**4. Synthesis of α-norbornenyl poly(ethylene oxide)-*bloc*-polyglycidol**

**a) Synthesis of α-norbornenyl poly(ethylene oxide):**

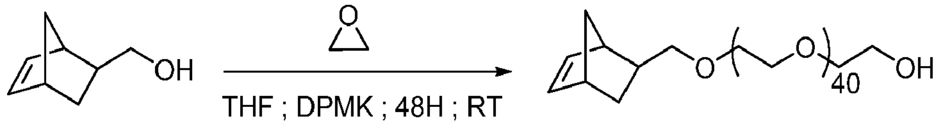


α-norbornenyl poly(ethylene oxide) was prepared by anionic ring-opening polymerization of ethylene oxide. 1.1 mL of norbornene methanol (1 eq.; $9.35\ 10^{-3}$ mol) were dissolved in 200 mL of THF previously cryodistilled. 11.7 mL of DPMK (0.8 eq.; 0.64 mol.$L^{-1}$) are added. Then 21 mL (45 eq.; 0.421 mol) of ethylene oxide stirred over sodium and cryodistilled were promptly added. The mixture was stirred during 48 hours under vacuum at room temperature,

and the anionic active centres were neutralized with 5 mL of acidic methanol. The polymer was precipitated in anhydrous diethyl ether, filtered, dissolved in dichloromethane, dried with $MgSO_4$, filtered on celite, concentrated, precipitated in diethyl ether, filtered and dried under vacuum.

- 50% yield
- $^1H$ NMR data in $CDCl_3$: $\delta$ (ppm) = 1.08-1.79 (m, 4H, $-CH_{2}$-$_{cycle}$); 2.72-3.45 (m, 3H,
- CH-$_{cycle}$); 3.63 (m, 164H, $-CH_2$-O-); 5.91-6.09 (m, 2H, -CH=CH-$_{cycle}$)
  $DP_{n;NMR}$=41 ; $M_n$;NMR=1930 g/mol
- SEC in THF: $M_{n;SEC}$=2240 g/mol (styrene eq) PDI=1.09

**b) Synthesis of $\alpha$-norbornenyl poly(ethylene oxide)-*bloc*-poly(glycidol actetal):**

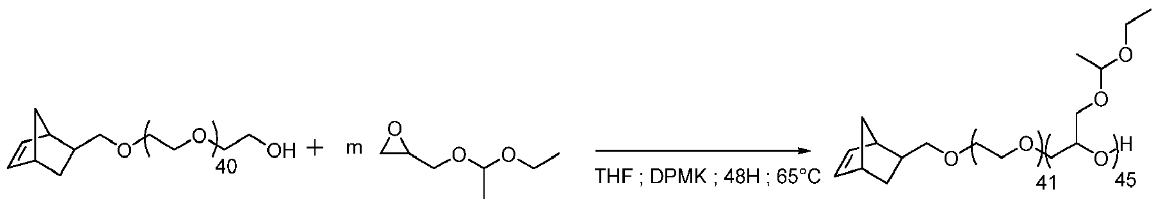


8 g of $\alpha$-norbornenyl poly(ethylene oxide) (1 eq; $M_n$=1930 g/mol; 4.14 $10^{-3}$ mol) were dissolved in 200 mL of freshly cryodistilled THF. Then, 5.2 mL of DPMK (0.8 eq; 0.64 mol/L) were added. Finally, 18 mL (30 eq.; 0.124 mol) of glycidol acetal are promptly added. The mixture was stirred during 48 hours under vacuum at 65°C, and the anionic active centres were neutralized with 5 mL of acidic methanol. The solvent was evaporated, the polymer was dissolved in dichloromethane, dried with $MgSO_4$, filtered on celite. The dichloromethane was evaporated. The polymer was dried under vacuum and lyophilized overnight in dioxane.

- 99% yield
- $^1H$ NMR data in $CDCl_3$: $\delta$ (ppm) = 1.1-1.5 (d, 278H, $-CH_3$); 2.72-3.45 (m, 3H, -CH-$_{cycle}$); 3.5-3.7 (m, 586H, $-CH_2$-O-, -CH-O-); 4.75 (s, 45H, -CH-); 5.91-6.09 (m, 2H, - CH=CH-$_{cycle}$)
  $DP_{n;PEO}$= 41; $DP_{n;PGLDAc}$= 45; $M_{n;NMR}$=8500g/mol
- SEC in THF: $M_n$=5930 g/mol (styrene eq) PDI=1.07

**c) Deprotection of $\alpha$-norbornenyl poly(ethylene oxide)-*bloc*-poly(glycidol actetal):**

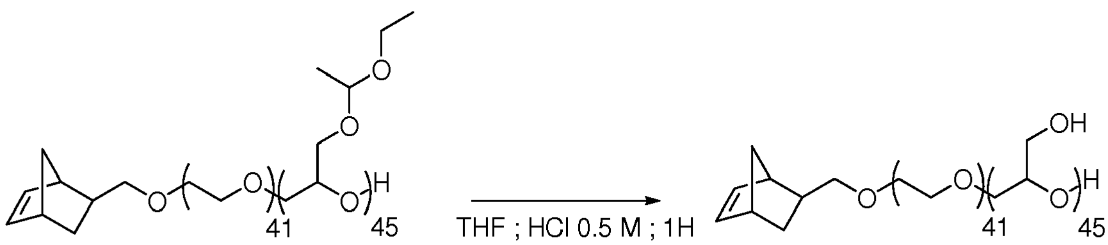


5 g of a-norbornenyl poly(ethylene oxide)-bloc-poly(glycidol actetal) were dissolved in 150 mL of THF. Then, 6.4 mL of HCl 37% were added. The reaction mixture was stirred during 1H at room temperature. Then, the solution was neutralized by adding $NaHCO_3$ saturated water solution. The solvent was evaporated; the polymer was dissolved in water, purified by ultrafiltration (MWCO 1000) and lyophilized overnight.

- 90% yield
- $^1H$ NMR data in DMSO-$d_6$: $\delta$ (ppm) = 2.72-3.45 (m, 3H, -CH-$_{cycle}$); 3.2-3.8 (m, 432H, $-CH_2$-O-, -CH-O-); 4.49 (s, 39H, -OH); 5.9-6.2 (m, 2H, -CH=CH-$_{cycle}$)
  $M_{n;NMR}$=5260 g/mol
- SEC in THF: $M_n$=2030 g/mol (styrene eq) PDI=1.03

**d) Acetal functionalization of α-norbornenyl poly(ethylene oxide)-*bloc*-polyglycidol**

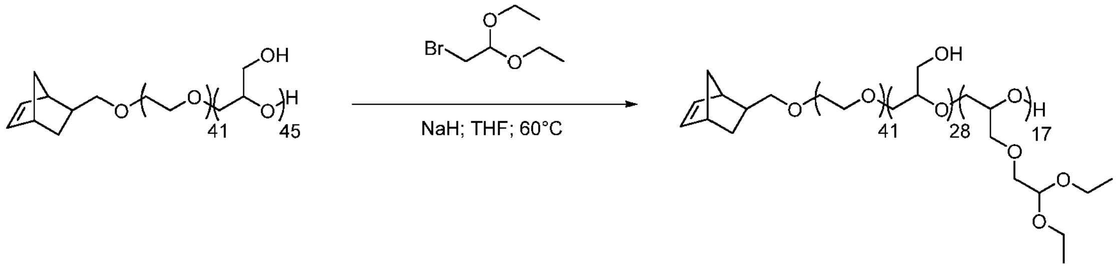


2.8 g ($M_n$=5260 g/mol; $DP_{n;PGLD}$=45; $n_{OH}$=2.4 $10^{-2}$ mol) of α-norbornenyl poly(ethylene oxide)-*bloc*-polyglycidol macromonomer was dissolved in 20 mL of freshly cryodistilled THF. 5 equivalents of NaH (M=24 g/mol; dispersed in mineral oil 60% (w/w)), previously washed with heptane to remove the mineral oil, were dispersed in 20 mL of THF. The macromonomer solution was added dropwise to NaH under stirring and under a nitrogen flux. After 30 min, 4.5 equivalents of bromoacetaldehyde diethyl acetal (M=197 $g.mol^{-1}$; d=1.31) were added dropwise. The reaction mixture was stirred for 12 hours at 60°C. NaH was then neutralized with a 3 N HCl solution and the solution mixture was evaporated, redissolved in $CH_2Cl_2$, dried with $MgSO_4$ and filtrated. Finally, $CH_2Cl_2$ was evaporated and the product was dried under vacuum and lyophilized overnight in dioxane.

- 87% yield
- $^1H$ NMR data in $D_2O$: δ (ppm) = 1.12-1.48 (m, 103H,-$CH_3$); 2.72-3.45 (m, 3H, -$CH_{-cycle}$); 3.3-4.0 (m, 532H, -$CH_2$-O-, -CH-O-); 5.9-6.2 (m, 2H, -CH=$CH_{-cycle}$)
- $DP_{n;PEO}$=41; $DP_{n:PGLD}$=28; $DP_{n;PGLDAc}$=17
- Functionalization: 38%
- $M_{n;NMR}$=7210 g/mol
- SEC in THF: $M_n$=3150 g/mol; PDI=1.3

**e) Synthesis of GS-functionalized α-norbornenyl-poly(ethylene oxide)-*bloc*-polyglycidol macromonomer**

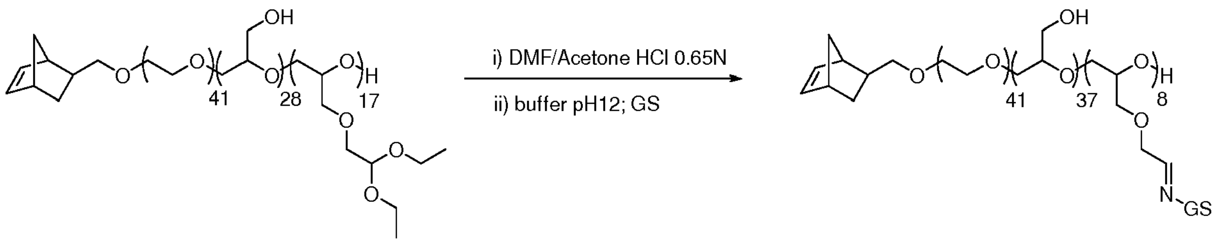


Acetal functionalized α-norbornenyl-poly(ethylene oxide)-*bloc*-polyglycidol macromonomer (2.36 g; $M_n$=7210 g/mol; $n_{Ac}$=5.56 $10^{-3}$ mol) was dissolved in 40 mL of 3M HCl solution. The mixture was stirring for 6 hours at room temperature. Then 280 mL of buffer solution pH 12 and NaOH pellets were added to basified the solution and finally, GS (5 eq.; M=477 g/mol; 13.20 g), dissolved in 70 mL of buffer solution pH 12, was added dropwise. The mixture was stirred during 12 hours at room temperature. After this time, the solvent was evaporated, the reminder taken up in a 10 mM solution of triethylamine in deionized $H_2O$ and purified by a 3 days dialysis using a 10 mM solution of triethylamine in deionized $H_2O$ as the solvent and a regenerated cellulose membrane (1 K Daltons) to separate the product from unreacted GS. The retained fraction was frozen with liquid nitrogen and lyophilized for 48 hours.

The structure was confirmed by $^1H$ NMR in $D_2O$

- 8 GS molecules per macromonomer
- Functionalization: 47%
- $M_{n;NMR}$=7640 g/mol

**f) Synthesis of polynorbornene-poly(ethylene oxide)-poly(ethylene oxide)-*bloc*-polyglycidol particles**

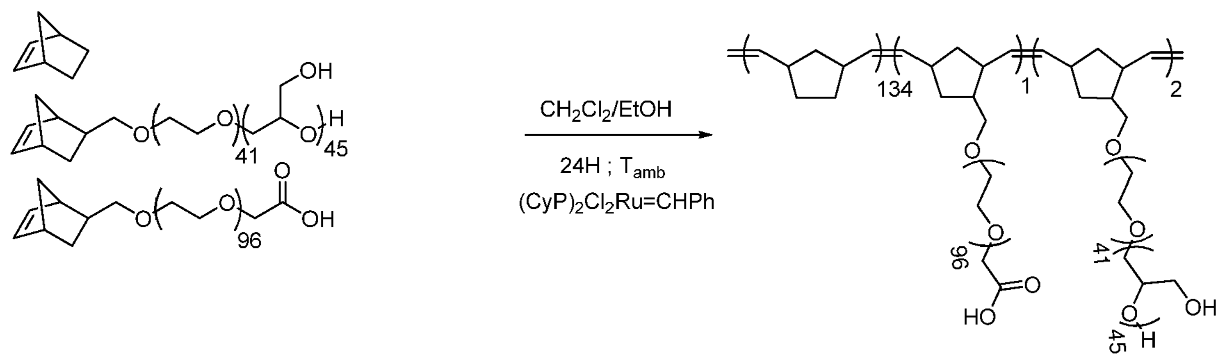


The Dispersion polymerization was carried out at room temperature under inert atmosphere (glovebox) and under stirring. Solvents were degassed according to the freeze-pump-thaw procedure. In a typical experiment, 30 mg ($3.6\ 10^{-5}$ mol) of Grubbs 1st generation complex were dissolved in 10 mL of dichloromethane/ethanol mixture (1:1 v/v). Norbornene (580 mg; $6.18\ 10^{-3}$ mol), α-norbornenyl-ω-carboxylic acid-poly(ethylene oxide) macromonomer (153 mg; $5.1\ 10^{-5}$ mol) and α-norbornenyl-poly(ethylene oxide)-*bloc*-polyglycidol macromonomer (582 mg; $1.1\ 10^{-4}$ mol) were first dissolved in 18 mL of dichlomethane/ethanol solution (35:65 v/v) and added to the catalyst. 0.2 mL of dodecane is also added as internal standard. The mixture was stirred during 24 hours. The desactivation of the reaction medium was performed by addition of 0.3 mL of ethyl vinyl ether. Then, the particles were transferred to DMF to carry out the grafting step onto titanium surfaces: first DMF was added dropwise, then dichloromethane and ethanol were evaporated under reduced pressure.

- Norbornene conversion: >99%
- Global macromonomer conversion: 92%
- Size distribution of the particles by DLS in the reaction medium ($EtOH/CH_2Cl_2$), in water and in DMF is given on Figure 4. In $EtOH/CH_2Cl_2$: D=245 nm (0.122). In $H_2O$: D=365 nm (0.358) (aggregation of the Particles). In DMF: D=230 nm (0.069).
- TEM observations of the particles are presented on Figure 5.

**g) Synthesis of polynorbornene-poly(ethylene oxide)-poly(ethylene oxide)-*bloc*-polyglycidol particles functionalized with GS**

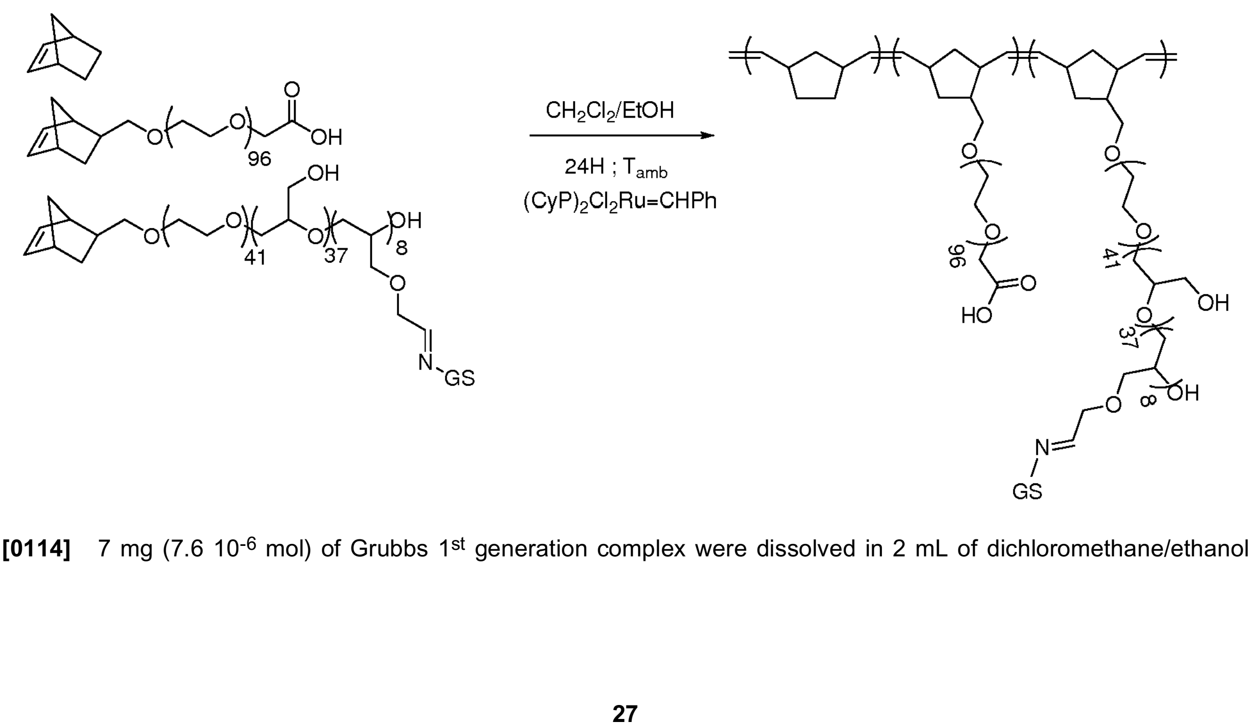


7 mg ($7.6\ 10^{-6}$ mol) of Grubbs 1st generation complex were dissolved in 2 mL of dichloromethane/ethanol

mixture (1:1 v/v). Norbornene (121 mg; 1.3 $10^{-3}$ mol), $\alpha$-norbornenyl-$\omega$-carboxylic acid-poly(ethylene oxide) macromonomer (32mg; 1.05 $10^{-5}$ mol) and $\alpha$-norbornenyl-poly(ethylene oxide)-*bloc*-polyglycidol macromonomer (182 mg; 2.4 $10^{-4}$ mol) were first dissolved in 6 mL of dichlomethane/ethanol solution (35:65 v/v) and added to the catalyst. 0.2 mL of dodecane is also added as internal standard. The mixture was stirred during 24 hours. The desactivation of the reaction medium was performed by addition of 0.2 mL of ethyl vinyl ether. Then, the particles were transferred to DMF to carry out the grafting step onto titanium surfaces:

- Norbornene conversion: >99%
- Size distribution of the particles by DLS in the reaction medium ($EtOH/CH_2Cl_2$), in water and in DMF is given on Figure 6. In $EtOH/CH_2Cl_2$: D=620 nm (0.30). In $H_2O$: D=565 nm (0.32). In DMF: D=520 nm (0.27)

**4. Activities of the compounds of the invention**

From a 24h bacterial culture, MRSA BCB8 were suspended in Mueller-Hinton broth to obtain a 0.5 McF suspension, which was diluted to a final concentration of $1.10^6$ $CFU.ml^{-1}$.

Then twofold serial dilutions of chemicals were prepared (from 256 $\mu g.ml^{-1}$ to 0.06 $\mu g.ml^{-1}$) and 100 $\mu l$ of MRSA BCB8 suspension were incubated with 200 $\mu l$ of chemical containing solutions for 24h at 37 °C.

After this time, suspension absorbances were measured at 600 nm. MICs were determined as the minimal concentration for which the lowest absorbance is observed.

The MICs were as follows:

MIC Vancomycin (Vanco.): 0.6 $\mu g.ml^{-1}$
MIC Macromonomer Vancomycin (Nb-PEO-Vanco; macro Vanco, as obtained by example 3.b)): 1.3 $\mu g.ml^{-1}$
MIC particles grafted with Vancomycin as obtained by example 3 c) (Vanco particles): 10.6 $\mu g.ml^{-1}$

The MICs are gathered in Figure 7 including also MICS measurements of Nb-PEO-OH (macro OH, equivalent to macro Vanco without Vancomycin), and Nb-PEO-OH particles (OH particles, equivalent to Vanco particles without Vancomycin).

## Claims

1. Polymer particles, the said particles being formed by polymer chains containing about 30 to 10000 monomer units, identical or different, derived from polymerization of monocyclic or polycyclic alkenes, wherein at least one of the said monomer units is substituted by a chain R comprising a polyethyleneglycol-polyglycidol chain of formula (I), wherein formula (I) is as follows:

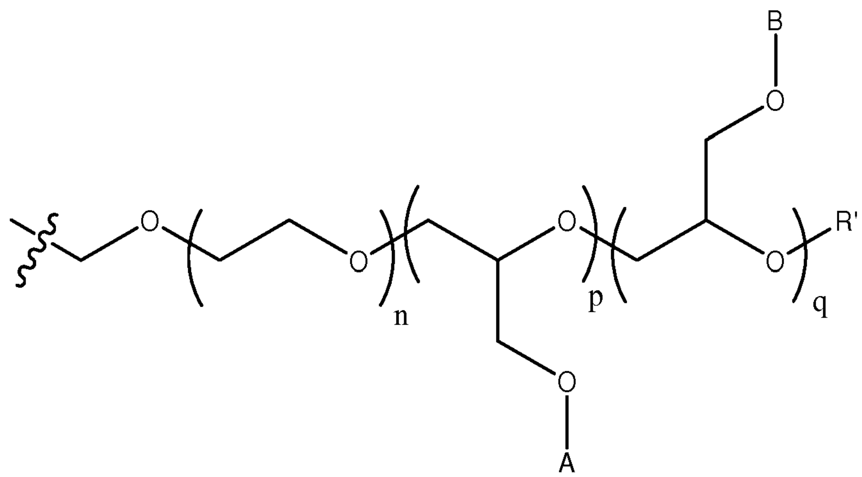

(I),

formula (I) wherein:

n represents an integer from about 0 to 300, especially from 10 to 100, p represents an integer from about 0 to 300, q represents an integer from about 0 to 300, with n+p+q is from about 10 to 300,
A represents a hydrogen atom or a group of the following formula (II):

-CONHAb1, where Ab1 represents an antibiotic with extracellular action,

B represents a hydrogen atom or a group of the following formula (III):

-CH2CNAb2, wherein Ab2 represents an antibiotic with intracellular action,

R' represents a hydrogen atom, -CH2CNAb2 or -CH2CNAb1 as defined above, with the proviso that when p is different from 0, then q is 0 and R' represents a hydrogen atom or -CONHAb1 as defined above, when q is different from 0, then p is 0 and R' represents a hydrogen atom or -CH2CNAb2, when p+q is not zero, at least one of the p or q moieties comprises the formula (II) or (III) respectively, and when said particles are formed by polymer chains with p+q is 0 exclusively, then at least one of said polymer chains presents a R chain comprising a polyethyleneglycol-polyglycidol chain of formula (I) where R' is -CONHAb1 as defined above,

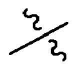

represents a covalent bond to which the polyethyleneglycol-polyglycidol chain is attached to the remainder of the R chain,

and wherein at least oneof said monomer units, identical or different from the monomer units substituted by R chain, is substituted by a group X, wherein X represents an alkyl or alkoxy chain with about 1 to 500 carbon atoms, preferably 40 to 400 carbon atoms, comprising a reactive function of the OH, halogen, NH2, C(O)X1 type, wherein X1 represents a hydrogen atom, a halogen atom, an OR" or NHR" group, in which R" represents a hydrogen atom or an alkyl group..

**2.** The polymer particles according to claim 1, wherein the monocyclic or polycyclic alkenes from which the monomer units are derived are selected from the group consisting of norbornene (bicyclo[2.2.1]hept-2-ene), tetracyclododecadiene, dicyclopentadiene, the dimer of norbornadiene, and cycloocta-1,5-diene.

**3.** The polymer particles according to claim 1 or 2, wherein the chain or chains R substituting the monomers are represented by the formula (I), more specifically wherein at least one, or all, of the following specific embodiments are fulfilled:

n+p+q is from 10 to 100; and/or
n is from 35 to 70, more specifically n is from 40 to 60 (e.g. n=45); and/or
either p or q is from 1 to 300.

**4.** The polymer particles according to anyone of claim 1-3, wherein the chain of formula (I) is of the following formula:

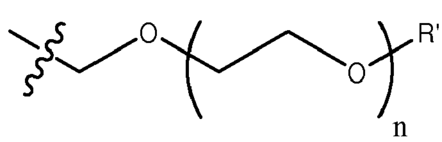
,

wherein R' -CONHAb1 and n is as defined in claim 1, and preferably n is from 1 to 300, more preferably from 10 to 100, or Ab1 is preferably vancomycin or a salt thereof.

**5.** The polymer particles according to anyone of claim 1-3, wherein R' in formula (I) is an hydrogen atom.

**6.** The polymer particles according to anyone of claim 1-5, wherein Ab1 represents a cephalosporin, including those from first to the fifth generations, such as cefalexin, cefuroxim, ceftriaxone, cefepime, ceftobiprole; a carbacephem, such as Loracarbef; a carbapenem, such as imipenem; a glycopeptide, such as vancomycin, teicoplanin or ramoplanin; a lipopeptide, such as daptomycin; a monobactam, such as aztreonam; a penicillin, such as amoxicillin; or a polymyxin, such as polymyxin B; preferably Ab1 is vancomycin and any salt thereof.

7. The polymer particles according to anyone of claim 1-6, wherein Ab2 represents an aminoglycoside, including gentamicin, neomycin, and streptomycin; an anzamycin, such as rifaximin; a lincosamide, such as clindamycin; a macrolide, such as azithromycin; a nitrofurane, such as furazolidone; an oxazolidinone, such as linezolid; a quinolone or a fluoroquinolone, such as nalidixic acid, ofloxacin, ciprofloxacin, or levofloxacin; a sulfonamide, such as sulfacetamide, furosemide; a tetracycline, such as doxycycline; preferably Ab2 is an aminoglycoside, preferably gentamicin or any salt thereof.

8. The polymer particles according to anyone of claim 1-7, wherein they comprise:

between about 0.5% and 99.5% of monomer units substituted by a chain R as defined in anyone of the preceding claims, the said chain R being identical for these monomers, and between about 0.5% and 99.5% of monomer units substituted by a chain R as defined in anyone of the preceding claims, the said chain R of these monomers being different from the chain R of the preceding monomers, more preferably said chain R comprises groups of formula (II) and the said other different chain R comprises groups of formula (III), and between 0.0% and about 99% of unsubstituted monomer units, optionally at least one of the monomer units substituted by a chain R is also substituted by a group X
and/or between about 0.5% and 99.5% of monomer units substituted by a chain R as defined in anyone of the preceding claims, the said chain R being identical or different for these monomers, and between about 0.5% and 99.5% of unsubstituted monomer units, optionally at least one of the monomer units substituted by a chain R is also substituted by a group X
and/or between about 0.5% and 99.5% of monomer units directly substituted by a group X as defined in anyone of the preceding claims, and between about 0.5% and 99.5% of monomer units substituted by a chain R as defined in anyone of the preceding claims, the said chain R being identical or different for these monomers, and between 0.0% and about 99.0% of unsubstituted monomer units,
the total of the percentages of the monomers mentioned above being 100%.

9. A monocyclic or polycyclic alkene based macro monomer of formula (IV) as follows:

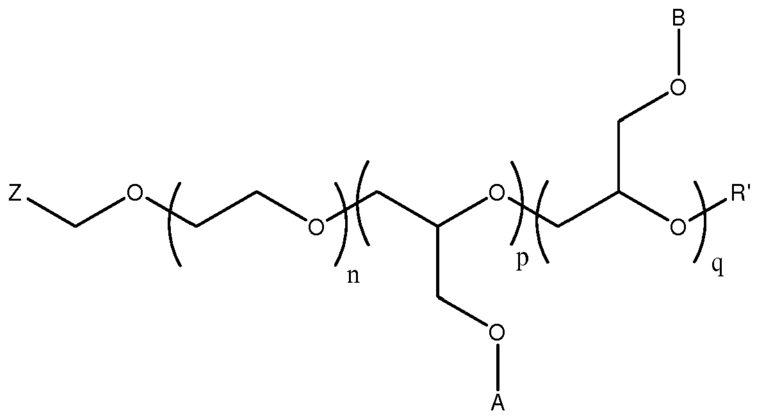

formula (IV) wherein
n represents an integer from about 0 to 300, especially from 10 to 100, p represents an integer from about 0 to 300, q represents an integer from about 0 to 300, with n+p+q is from about 10 to 300,
A represents a hydrogen atom or a group of the following formula (II):

-CONHAb1, where Ab1 represents an antibiotic with extracellular action,

B represents a hydrogen atom or a group of the following formula (III):

-CH2CNAb2, wherein Ab2 represents an antibiotic with intracellular action,

R' represents a hydrogen atom, -CH2CNAb2 or -CONHAb1 as defined above, with the proviso that when p is different

from 0, then q is 0 and R' represents a hydrogen atom or -CONHAb1, when q is different from 0, then p is 0 and R' represents a hydrogen atom or -CONHAb2, when p+q is not zero, at least one of the p or q moieties comprises the formula (II) or (III) respectively, and when p+q is 0, then R' can be -CONHAb1 only,
Z represents a monocyclic or polycyclic alkene to which the polyethyleneglycol-polyglycidol chain is attached, optionally substituted by a group X, wherein X represents an alkyl or alkoxy chain with about 1 to 500 carbon atoms, preferably 40 to 400 carbon atoms, comprising a reactive function of the OH, halogen, NH2, C(O)X1 type, wherein X1 represents a hydrogen atom, a halogen atom, an OR" or NHR"group, in which R" represents a hydrogen atom or an alkyl group.

**10.** The macromonomer according to claim 9, wherein it is of the following formula (V):

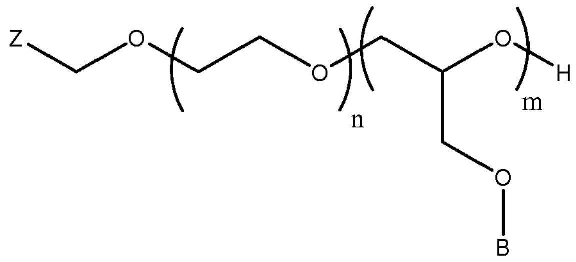

in which Z is as defined in claim 9, n is as defined in anyone of the preceding claims, more preferably is 0, and m is q as defined in anyone of the previous claims, and B is as defined in anyone of the previous claims, wherein at least one of the m moieties comprises the formula (III).

**11.** The macromonomer according to anyone of claim 9-10, wherein the cyclic alkene is selected from norbornene, tetracyclododecadiene, dicyclopentadiene, the dimer of norbornadiene, and cycloocta-1,5-diene, more particularly it is norbornene.

**12.** A biomaterial comprising a support material having on its support surface covalently bonded polymer particles as defined in anyone of claims 1-8.

**13.** The biomaterial according to claim 12, wherein the support material is chosen from:

- metals or metal oxides thereof, preferably titanium or TiO2,
- metal alloys, in particular alloys with or without shape memory such as Ni-Ti alloys,
- polymers, such as polyethylene terephthalate (PET), polytetrafluoroethylene (PTFE), polyvinylidine fluoride (PVDF), polyether etherketone (PEEK), polycarbonate-urethane (PCU), polyhydroxyethylmethacrylate (PHEMA), polymethylmethacrylate (PMMA), poluethylmethacrylate (PEMA), poly(4-hydroxystyrene),
- copolymers, such as the copolymer ethylene vinyl acetate (EVA), the copolymer vinylidene fluoride-hexafluoropropylene P(VDF-HFP), poly(lactic acid)-co-poly(glycolic acid) (PLA-PGA), copolymers of polymethylmethacrylate (PMMA) and poluethylmethacrylate (PEMA),
- ceramics, such as hydroxyapatites, or compounds of hydroxyapatites and tricalcium phosphate.

**14.** A medical device, including implants, prostheses, stents, lenses or cements as well as any pharmaceutical composition, comprising a biomaterial as defined in anyone of claim 12 and 13.

**15.** The medical device of claim 14, wherein the medical device is an implant, prostheses, stents, lenses, cements, or any pharmaceutical composition.

**16.** Polymer particles of anyone of claims 1-8, a biomaterial of anyone of claims 12-13, or a medical device according to anyone of claim 14-15, as a medicine, more particularly for a use in the treatment of bacterial infections.

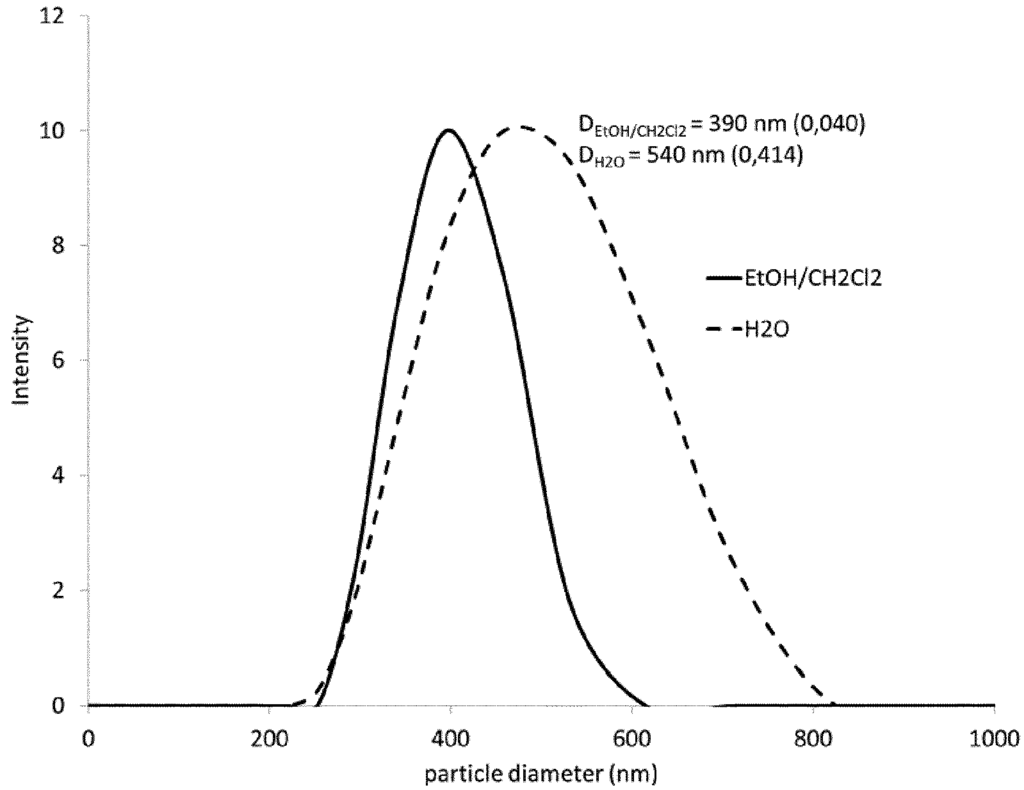


Figure 1

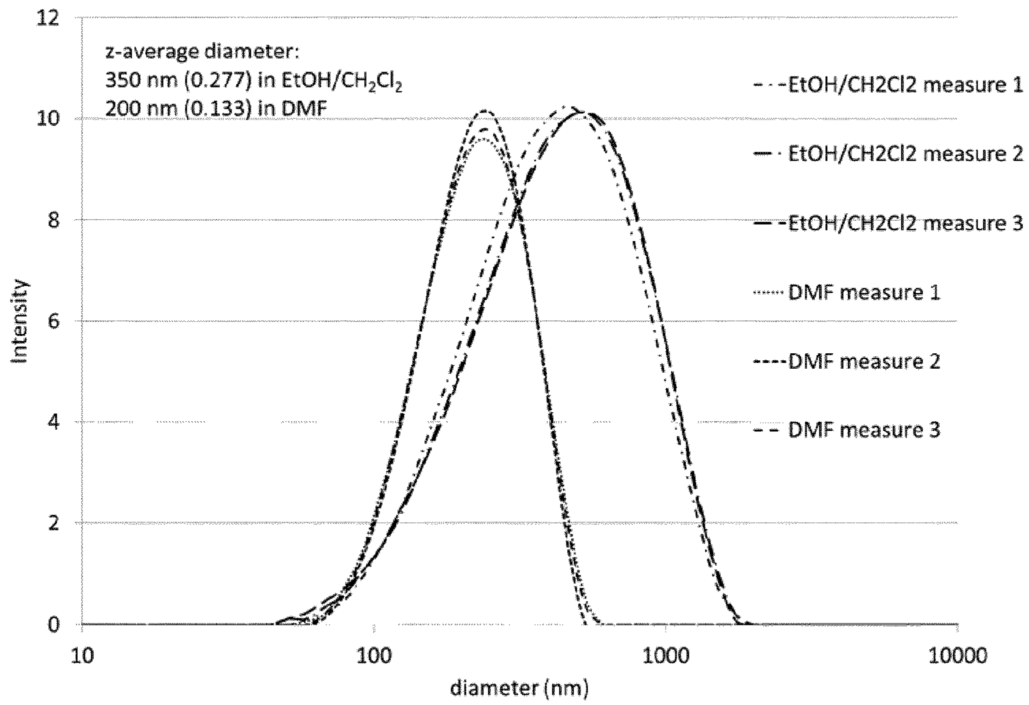


Figure 2

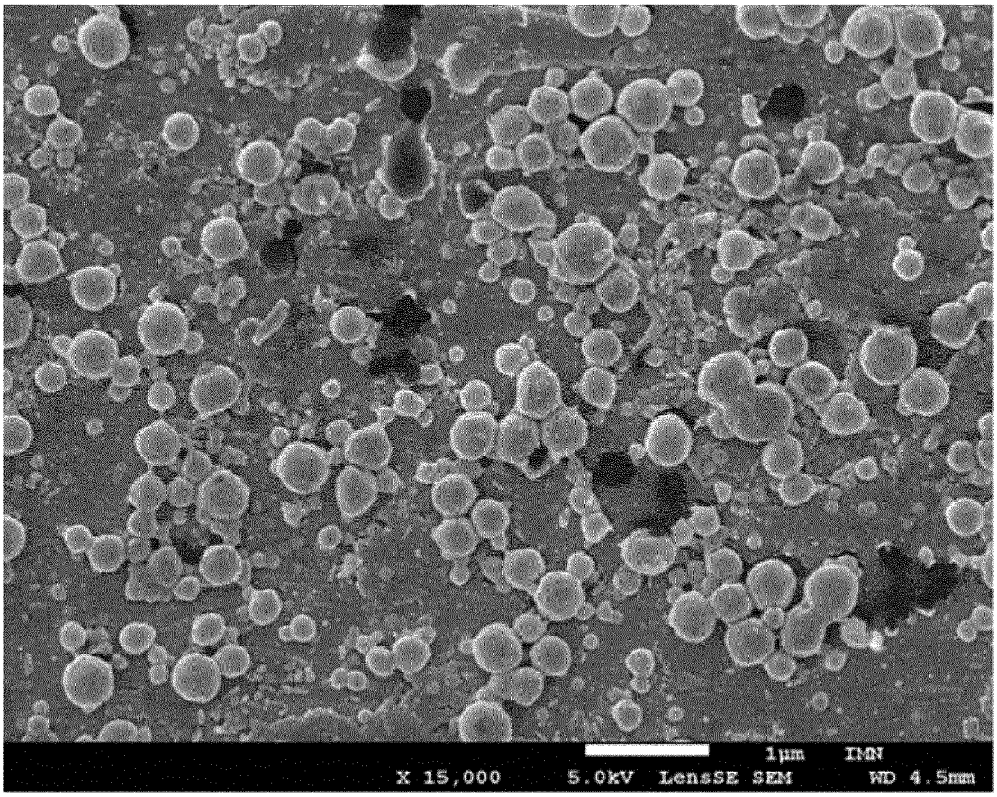


Figure 3

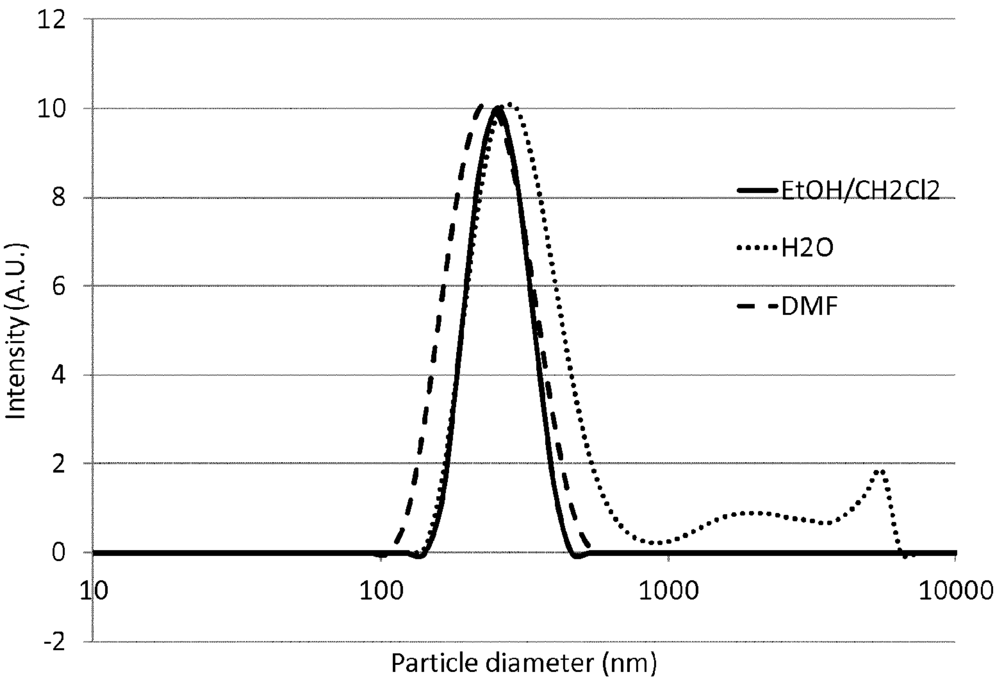


Figure 4

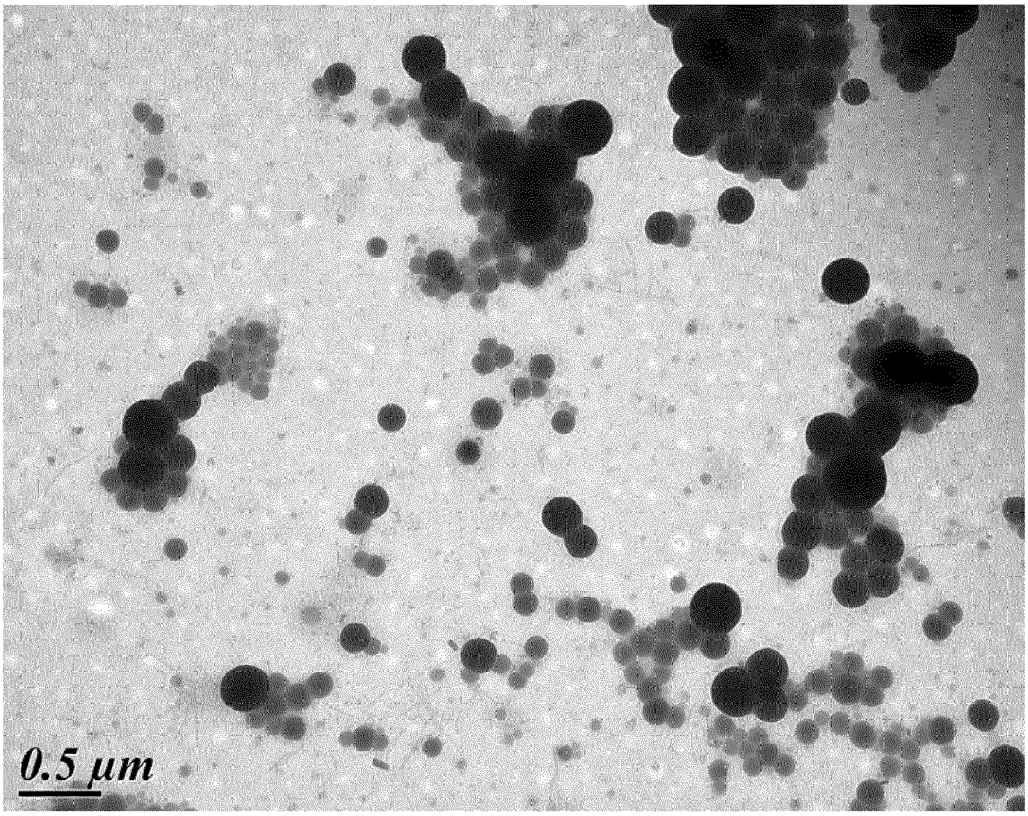


Figure 5

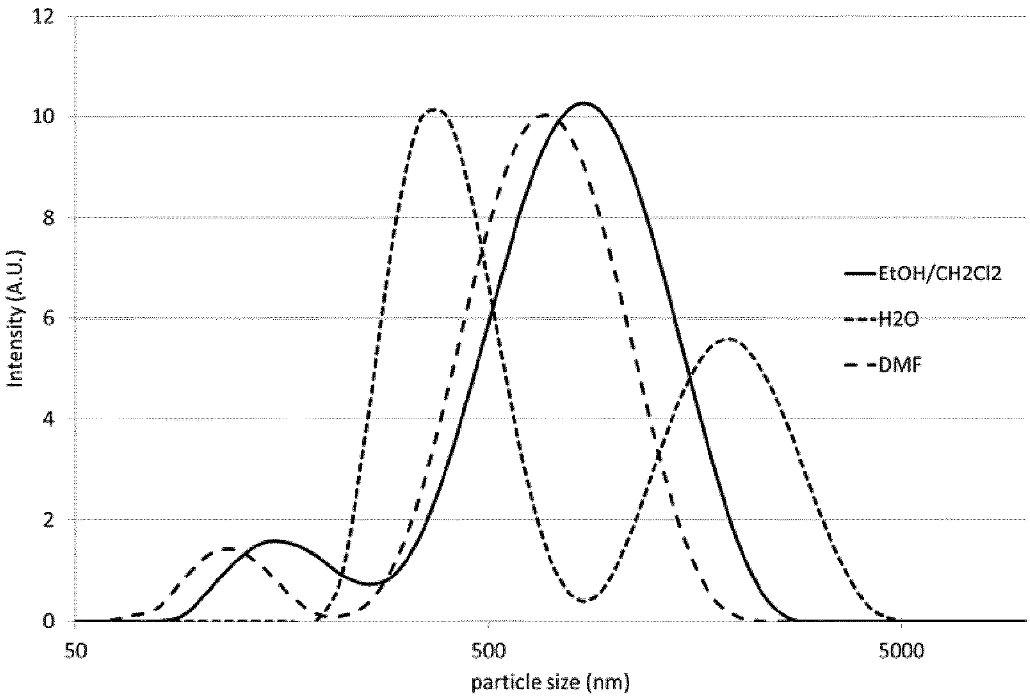


Figure 6

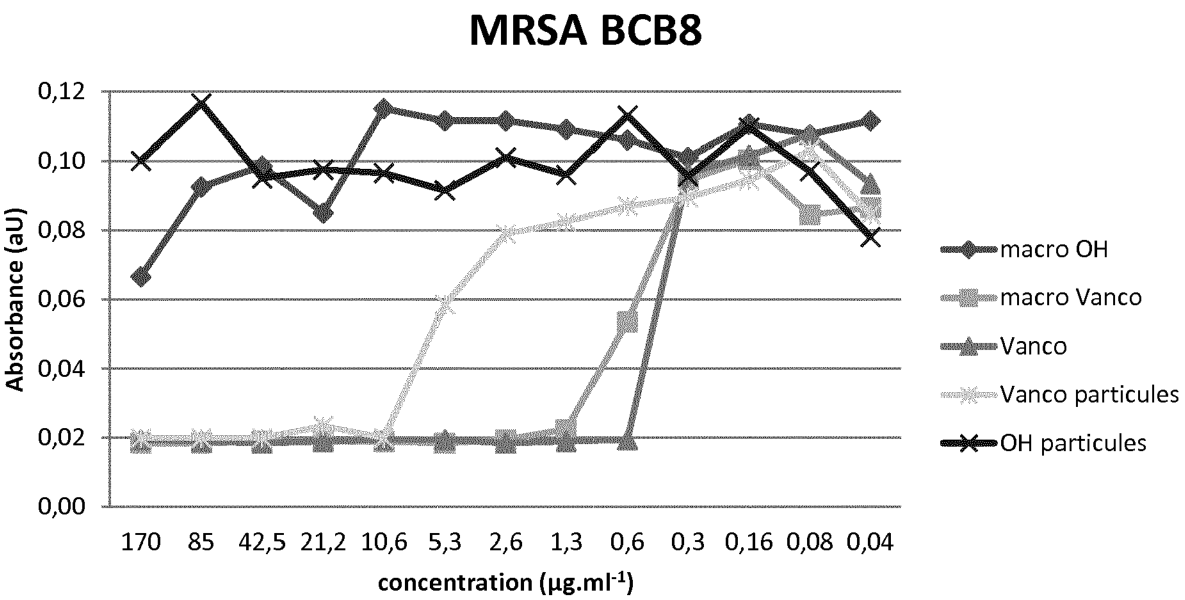
MRSA BCB8
Absorbance (aU)
0,12
0,10
0,08
0,06
0,04
0,02
0,00
170
85
42,5
21,2
10,6
5,3
2,6
1,3
0,6
0,3
0,16
0,08
0,04
concentration (µg.ml-1)
macro OH
macro Vanco
Vanco
Vanco particules
OH particules

Figure 7

Europäisches Patentamt
European Patent Office
Office européen des brevets

# EUROPEAN SEARCH REPORT

Application Number
EP 14 30 7083

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br><br><br>A | EP 1 758 621 B1 (CENTRE NAT RECH SCIENT [FR]; INST NAT SANTE RECH MED [FR]; UNIV BORDEA) 11 August 2010 (2010-08-11)<br>* examples a-c *<br>* page 19, paragraph [0066] *<br>* page 16, paragraph [0052] *<br>* Schéma 2;<br>page 22 *<br>* Schéma de synthèse;<br>page 19 *<br>* page 2, paragraph [0002] - paragraph [0006] *<br>* claims 1-18 *<br>----- | 1-4,6-9, 11-16<br><br><br>5,10 | INV.<br>C08G65/22<br>A61K47/48<br>C08F32/04 |
| A,D | EP 1 771 492 B1 (CENTRE NAT RECH SCIENT [FR]; INST NAT SANTE RECH MED [FR]; UNIV BORDEA) 10 November 2010 (2010-11-10)<br>* examples A-C *<br>* claims 1-23 *<br>----- | 1-16 | |
| A | MINH NGOC NGUYEN ET AL: "Impact of RGD Nanopatterns Grafted onto Titanium on Osteoblastic Cell Adhesion",<br>BIOMACROMOLECULES,<br>vol. 13, no. 3,<br>28 February 2012 (2012-02-28), pages 896-904, XP055179890,<br>ISSN: 1525-7797, DOI: 10.1021/bm201812u<br>* Scheme 1 *<br>* table 2 *<br>----- | 1-16 | TECHNICAL FIELDS SEARCHED (IPC)<br>C08G<br>A61K<br>C08F |
| A | EP 2 726 107 A1 (CENTRE NAT RECH SCIENT [FR]; UNIV POITIERS [FR]; INST NAT SANTE RECH M) 7 May 2014 (2014-05-07)<br>* claims 1-19 *<br>* examples 1-17 *<br>-----<br>-/-- | 1-16 | |

1

The present search report has been drawn up for all claims

| Place of search | Date of completion of the search | Examiner |
|---|---|---|
| The Hague | 13 May 2015 | Popescu, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 30 7083

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MIKI K ET AL: "Ring-opening metathesis polymerization-based synthesis of polymeric nanoparticles for enhanced tumor imaging in vivo: Synergistic effect of folate-receptor targeting and PEGylation", BIOMATERIALS, ELSEVIER SCIENCE PUBLISHERS BV., BARKING, GB, vol. 31, no. 5, 23 October 2009 (2009-10-23), pages 934-942, XP026790423, ISSN: 0142-9612 [retrieved on 2009-10-23] * Scheme 1 * ----- | 1-16 | |
| | | | TECHNICAL FIELDS SEARCHED (IPC) |

1

The present search report has been drawn up for all claims

| Place of search | Date of completion of the search | Examiner |
|---|---|---|
| The Hague | 13 May 2015 | Popescu, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 30 7083

This annex lists the patent family members relating to the patent documents cited in the above-mentioned European search report. The members are as contained in the European Patent Office EDP file on
The European Patent Office is in no way liable for these particulars which are merely given for the purpose of information.

13-05-2015

| Patent document cited in search report | Publication date | Patent family member(s) | Publication date |
|---|---|---|---|
| EP 1758621 B1 | 11-08-2010 | AT 477007 T | 15-08-2010 |
| | | EP 1758621 A1 | 07-03-2007 |
| | | ES 2350527 T3 | 24-01-2011 |
| | | FR 2871701 A1 | 23-12-2005 |
| | | JP 5383039 B2 | 08-01-2014 |
| | | JP 2008503553 A | 07-02-2008 |
| | | US 2008004398 A1 | 03-01-2008 |
| | | WO 2006008386 A1 | 26-01-2006 |
| EP 1771492 B1 | 10-11-2010 | AT 487750 T | 15-11-2010 |
| | | EP 1771492 A1 | 11-04-2007 |
| | | ES 2360470 T3 | 06-06-2011 |
| | | FR 2871803 A1 | 23-12-2005 |
| | | JP 5265914 B2 | 14-08-2013 |
| | | JP 2008503633 A | 07-02-2008 |
| | | US 2009123555 A1 | 14-05-2009 |
| | | WO 2006008387 A1 | 26-01-2006 |
| EP 2726107 A1 | 07-05-2014 | EP 2726107 A1 | 07-05-2014 |
| | | FR 2977162 A1 | 04-01-2013 |
| | | US 2014219925 A1 | 07-08-2014 |
| | | WO 2013001244 A1 | 03-01-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1771492 A
- EP 1758621 A